# EUROPEAN PATENT SPECIFICATION

(11) **EP 3 756 657 B1**
(45) Date of publication and mention of the grant of the patent: **05.03.2025**
(21) Application number: 20164025.7
(22) Date of filing: 23.12.2010
(51) Int. Cl.: A61K 9/48, A61K 38/28, A61P 3/10, A61M 37/00, A61M 5/142, A61B 1/07, A61J 7/00, A61B 5/145, A61B 5/06, A61B 5/00, A61M 31/00, A61B 5/07, A61P 5/14, A61P 19/10, A61P 35/00

(54) **SWALLOWABLE DRUG DELIVERY DEVICE**
VORRICHTUNG ZUR FREISETZUNG EINES SCHLUCKBAREN WIRKSTOFFS
DISPOSITIF D'ADMINISTRATION DE MÉDICAMENTS À AVALER

(30) Priority: 24.12.2009 US 28476609 P; 15.03.2010 US 34033110 P; 10.05.2010 US 39530410 P
(43) Date of publication of application: 30.12.2020
(62) Divisional of application: 18191328.6
(73) Proprietor: Rani Therapeutics, LLC, San Jose, CA 95131 (US)
(72) Inventor: IMRAN, Mir, Los Altos Hills, CA 94022 (US)
(74) Representative: Forrest, Stuart

(56) References cited:
- WO-A1-03/068061
- WO-A1-2009/041525
- WO-A2-2004/058041
- US-A1- 2004 253 304
- US-A1- 2005 267 414
- US-A1- 2008 255 543
- US-A1- 2010 179 381

## Description

### BACKGROUND OF THE DISCLOSURE

Field of the disclosure. Embodiments of the invention relate to swallowable drug delivery devices. More specifically, embodiments of the invention relate to swallowable drug delivery devices for delivering drugs to the small intestine.

While there has been an increasing development of new drugs in recent years for the treatment of a variety of diseases, many have limited application because they cannot be given orally. This is due to a number of reasons including: poor oral toleration with complications including gastric irritation and bleeding; breakdown/degradation of the drug compounds in the stomach; and poor, slow or erratic absorption of the drug. Conventional alternative drug delivery methods such as intravenous and intramuscular delivery have a number of drawbacks including pain and risk of infection from a needle stick, requirements for the use of sterile technique and the requirement and associated risks of maintaining an IV line in a patient for an extended period of time. While other drug delivery approaches have been employed such as implantable drug delivery pumps, these approaches require the semi-permanent implantation of a device and can still have many of the limitations of IV delivery. Thus, there is a need for an improved method for delivery of drugs and other therapeutic agents.
The prior art document US 2004/0253304 A1 relates to a drug administration apparatus consisting of an ingestible capsule comprising a drug stored in powder form within the capsule, a sensor that changes in state in response to disposition of the capsule, a hydrophilic membrane, and a driving mechanism for driving the drug directly through an endothelial layer of the gastrointestinal tract in response to the change in state of the sensor. The hydrophilic membrane allows fluid from the gastrointestinal tract to enter into the capsule prior to activation of the driving mechanism.

US2008/255543 discloses a body-insertable apparatus for introducing into a subject to inject medical agent stored in a casing into a desired part in the subject. The body-insertable apparatus includes a fixing unit which fixes the casing to the desired part, and a projecting unit which projects, from the casing, an injection needle that is intended to inject the medical agent. The fixing unit and the projecting unit are driven by a driving source.

WO2004/058041 discloses an *in vivo* sensing device which includes an immobilizer that may immobilize the device in an *in vivo* location. The immobilizer may be activated by for example a processor or in response to an *in vivo* condition or in response to a signal from an outside operator.

WO2009/041525 discloses an introduction-into-subject system which is capable of making a puncture of a needle in a layer subjected to puncture. A capsule endoscope is disclosed, having a needle which can project from the surface of a casing and can be housed under the surface and the capsule endoscope has a permanent magnet. A magnetic field control part is intended to control a magnetic field generation part to generate a magnetic field which varies the direction of the permanent magnet on the basis of the magnetization direction of the permanent magnet at the capsule endoscope, the position of the needle at the capsule endoscope, and the direction of the tip of the needle.

WO03/068061 discloses a device that is used for the delivery of substances and for intracorporeal sampling through the ingestion of a capsule which is particularly intended for the intestine of a patient or animal. The capsule is disclosed as consisting of a body comprising an energy source, an emitter/receiver, a means of taking physical measurements in relation to the environment and position of the body, a module for delivering substances and/or a sampling module. All of these components are intended to be controlled by control means.

US2005/267414 discloses a device and method that is intended for releasing a therapeutic agent, wherein the device is for releasing a therapeutic agent brought into a position for therapy in the interior of a patient's body via a probe, comprising: a release, assigned to the probe and movable from a prestressing position into a destressing position, for releasing the therapeutic agent.

### BRIEF SUMMARY OF THE DISCLOSURE

Examples of the disclosure provide devices, systems, kits and methods for delivering drugs and other therapeutic agents to various locations in the body. Many examples provide a swallowable device for delivering drugs and other therapeutic agents within the Gastrointestinal (GI) tract. Particular examples provide a swallowable device such as a capsule for delivering drugs and other therapeutic agents into the wall of the small intestine, large intestine or other GI organ wall. Examples of the invention are particularly useful for the delivery of drugs and other therapeutic agents which are poorly absorbed, poorly tolerated and/or chemically degraded (e.g. breakdown of the chemical structure of the molecule) within the GI tract (e.g. the digestive enzymes and acids in the stomach). Further, examples of the disclosure can be used to deliver drugs which were previously only capable of or preferably delivered by intravenous or other form of parenteral administration (e.g., intramuscular, etc). Additionally, examples of the disclosure are useful for achieving rapid release of a drug into the blood stream via oral delivery.

Examples of the disclosure provide devices, systems, kits and methods for delivering drugs and other therapeutic agents to various locations in the body. Many examples provide a swallowable device for delivering drugs and other therapeutic agents within the Gastrointestinal (GI) tract. Particular examples provide a swallowable device such as a capsule for delivering drugs and other therapeutic agents into the wall of the small intestine, large intestine or other GI organ wall. Examples of the disclosure are particularly useful for the delivery of drugs and other therapeutic agents which are poorly absorbed, poorly tolerated and/or chemically degraded (e.g. breakdown of the chemical structure of the molecule) within the GI tract/ Further, examples of the disclosure can be used to deliver drugs which were previously only capable of or preferably delivered by intravenous or other form of parenteral administration (e.g., intramuscular, etc).

### SUMMARY OF THE INVENTION

According to the present invention there is provided an ingestible device comprising the features of claim 1. Preferred embodiments of the present invention are defined in the dependent claims.

In one aspect of the invention, the actuator includes a release element comprising a material configured to degrade upon exposure to a selected pH in the gastrointestinal tract such that upon degradation, the preparation is advanced into the lumen wall. For example, the selected pH is greater than about 7.4. In an alternative aspect, the actuator comprises a spring (80), the release element being coupled to the spring to retain the spring in a compressed state (85) and release the spring upon degradation of the release element.

In another aspect of the invention, the therapeutic agent preparation includes at least a first therapeutic agent and a second therapeutic agent.

In yet a further aspect of the invention, the therapeutic agent preparation comprises a therapeutically effective dose of insulin for the treatment of diabetes or a glucose regulation disorder.

In yet a further aspect of the invention, the therapeutic agent preparation comprises a therapeutically effective dose of an incretin for the treatment of diabetes or a glucose regulation disorder. For example, wherein the incretin comprises a glucagon like peptide-1 (GLP-1), a GLP-1 analogue, exenatide, liraglutide, albiglutide, taspoglutide or a gastric inhibitory polypeptide (GIP).

In yet a further aspect of the invention, the therapeutic agent preparation comprises a combination of therapeutic agents for the treatment of diabetes or a glucose regulation disorder, optionally, wherein the combination comprises a therapeutically effective dose of an incretin and a therapeutically effective dose of a biguanide. For example, the incretin comprises exenatide and the biguanide comprises metformin, or the dosages of the incretin and the biguanide are matched to produce an improved level of blood glucose control for an extended period.

In yet a further aspect of the invention, the therapeutic agent preparation comprises a therapeutically effective dose of growth hormone, a therapeutically effective dose of parathyroid hormone for the treatment of osteoporosis or a thyroid disorder, a therapeutically effective dose of a chemotherapeutic agent for the treatment of cancer, a therapeutically effective dose of antibiotic, a therapeutically effective dose of an antiviral compound, optionally wherein the antiviral compound comprises a protease inhibitor, or a therapeutically effective does of an anti-seizure compound, optionally wherein the anti-seizure compound comprises furosemide.

Further details of these and other embodiments and aspects of the invention are described more fully below, with reference to the attached drawing figures.

### BRIEF DESCRIPTION OF THE DRAWINGS

Fig. 1a is a lateral viewing showing an embodiment of a swallowable drug delivery device.
Fig. 1b is a lateral viewing showing an embodiment of a system including a swallowable drug delivery device.
Fig. 1c is a lateral viewing showing an embodiment of a kit including a swallowable drug delivery device and a set of instructions for use.
Fig. 1d is a lateral viewing showing an embodiment of a swallowable drug delivery device including a drug reservoir.
Fig. 2 is a lateral view illustrating an embodiment of the swallowable drug delivery device having a spring loaded actuation mechanism for advancing tissue penetrating members into tissue.
Fig. 3 is a lateral view illustrating an embodiment of the swallowable drug delivery device having a spring loaded actuation mechanism having a first motion converter.
Fig. 4 is a lateral view illustrating an embodiment of the swallowable drug delivery device having a spring loaded actuation mechanism having first and a second motion converter.
Fig. 5 is a perspective view illustrating engagement of the first and second motion converters with the tissue penetrating member and delivery members.
Fig. 6 is a cross sectional view illustrating an embodiment of the swallowable drug delivery device having a single tissue penetrating member and an actuating mechanism for advancing the tissue penetrating member.
Fig. 7a is a cross sectional view illustrating an embodiment of the swallowable drug delivery device having a multiple tissue penetrating members and an actuating mechanism for advancing the tissue penetrating members.
Fig. 7b is a cross sectional view illustrating deployment of the tissue penetrating members of the embodiment of Fig. 7a to deliver medication to a delivery site and anchor the device in the intestinal wall during delivery.
Figs. 8a-8c are side view illustrating positioning of the drug delivery device in the small intestine and deployment of the tissue penetrating members to deliver drug; Fig. 8a shows the device in the small intestine prior to deployment of the tissue penetrating members with the release element in tact; Fig. 8b shows the device in the small intestine with the release element degraded and the tissue penetrating elements deployed; and Fig. 8c shows the device in the small intestine with the tissue penetrating elements retracted and the drug delivered.
Fig. 9a shows an embodiment of a swallowable drug delivery device including a capsule having bio-degradable seams positioned to produce controlled degradation of the capsule in the GI tract.
Fig. 9b shows the embodiment of Fig. 9a after having been degraded in the GI tract into smaller pieces.
Fig. 10 shows an embodiment of a capsule having biodegradable seams including pores and/or perforations to accelerate biodegradation of the capsule.
Fig. 11 is a lateral viewing illustrating use of an embodiment of a swallowable drug delivery device including transit of device in the GI tract and operation of the device to deliver drug.

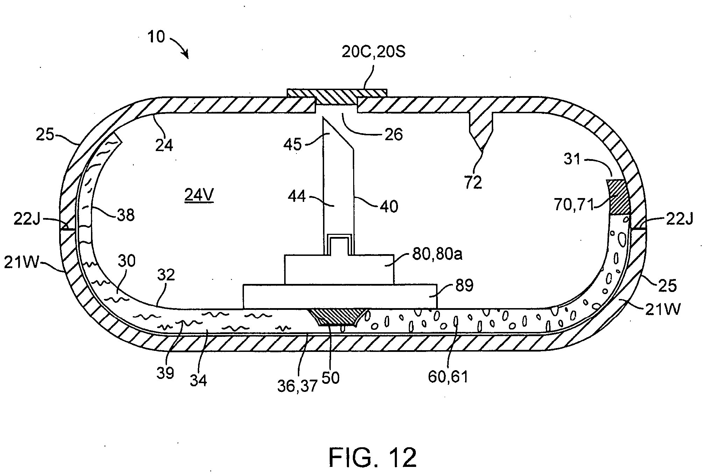

Fig. 12 is a lateral cross sectional view illustrating an embodiment of the swallowable drug device having an expandable member such as an expandable balloon.

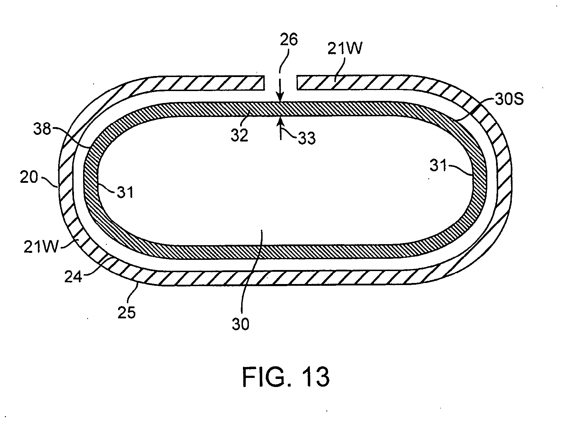

Fig. 13 is a lateral view illustrating an embodiment of an expandable balloon in an inflated state inside an embodiment of the swallowable capsule
Fig. 14a-14c are lateral views illustrating inflation of the expandable balloon using chemical reactants, Fig. 14a shows the balloon in a non-inflated state with the separation valve closed; Fig. 14b shows the balloon with valve open and mixing of the chemical reactants; and Fig. 14c shows the balloon in an inflated state.
Fig. 15 shows an embodiment of a separation valve having pinching features.

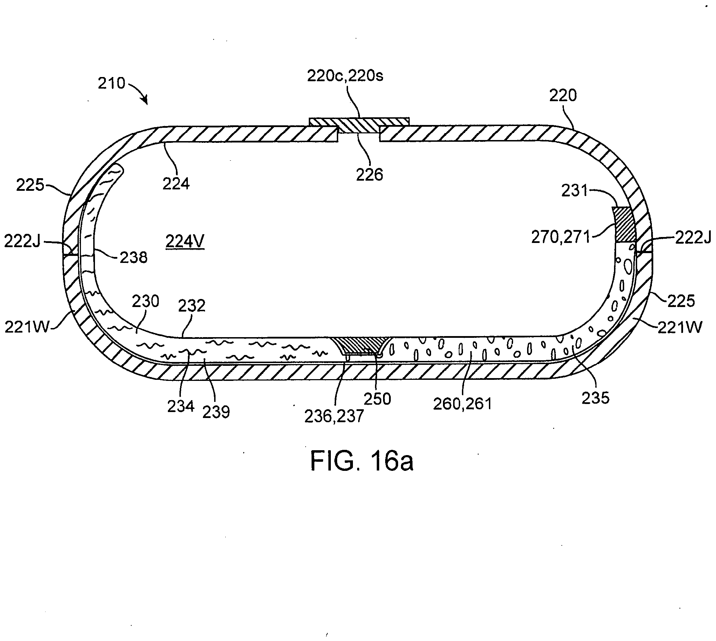

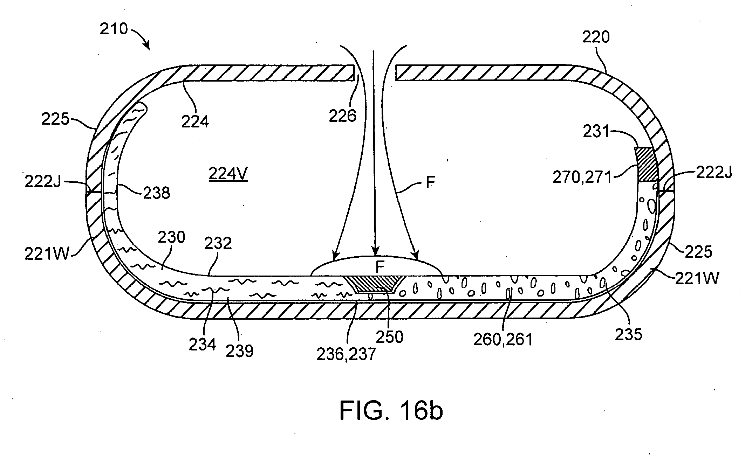

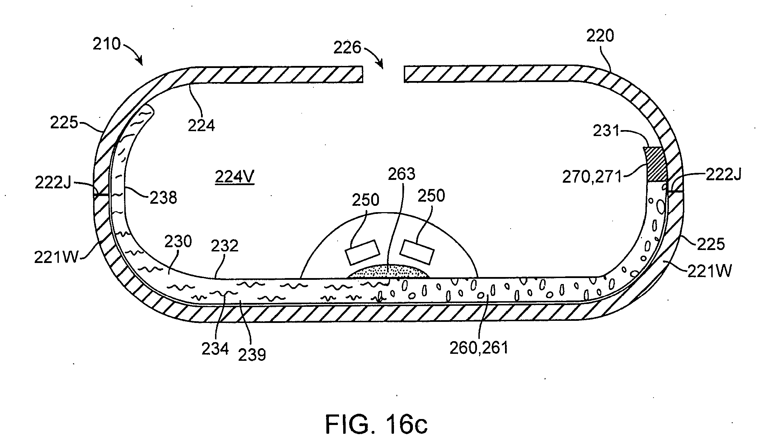

Fig. 16a-16c are lateral views illustrating use of a swallowable drug delivery device having a biodegradable coated capsule coating and a biodegradable separation valve to initiate inflation of the balloon in the small intestine. Fig. 16a shows the balloon in a non-inflated state with the capsule coating intact and the separation valve closed; Fig. 16b shows the capsule coating degraded and resulting ingress of intestinal fluid into the capsule interior to make contact with the isolation valve; and Fig. 16c shows the degradation and opening of the isolation valve from contact with intestinal fluid.
Fig. 17a shows is cross sectional view of an embodiment of a separation valve having a beam like structure.
Fig. 17b shows is a top view of the embodiment of Fig. 17a.
Fig. 18 shows an embodiment of a separation valve comprising a collar valve
Figs. 19a and 19b show an embodiment of the expandable balloon having a deflation valve comprising a biodegradable section of the balloon wall.
Fig. 20a is a side view of an embodiment of the tissue penetrating member.
Fig. 20b is a bottom view of an embodiment of the tissue penetrating member illustrating placement of the tissue retaining features.
Fig. 20c is a side view of an embodiment of the tissue penetrating member having a separate drug containing section.
Fig. 21a is a lateral view showing use of an advancement member to couple the tissue penetrating member to the expandable balloon.
Fig. 21b is a bottom view showing an embodiment of an advancement member having a larger surface area than the tissue penetrating member so as to function as a force concentrating element.
Fig. 22a is a lateral view showing use of an advancement member and an underlying platform to couple one or more tissue penetrating members to the expandable balloon.
Fig. 22b is a lateral view showing an embodiment of a platform having multiple advancement members and tissue penetrating members.
Figs. 23a and 23b are lateral views illustrating use of an embodiment of a swallowable device having platforms and tissue penetrating members placed on opposite sides of the balloon to achieve bilateral deployment of the tissue penetrating members; Fig. 23a shows the balloon in a non inflated state and Fig. 23b shows the balloon inflated with the penetrating members deployed.
Figs. 24a and 24b are cross sectional views illustrating use of an embodiment of a swallowable device having tissue penetrating members distributed around the entire perimeter of the balloon; Fig. 24a shows the balloon in a non inflated state and Fig. 24b shows the balloon inflated and the penetrating members placed in a distributed pattern within the intestinal wall.
Figs. 25a and 25b are lateral views illustrating use of an embodiment of a swallowable device having drug reservoirs compressible by expansion of the inflatable balloon; Fig. 25a shows the balloon in a non inflated state, and Fig. 25b shows the balloon inflated with drug injected from the reservoir into the intestinal wall.
Fig. 26 is a lateral view illustrating an embodiment of a manifold for coupling two or more drug reservoirs to a hollow tissue penetrating member.
Figs. 27a and 27b shows an embodiment of a colar type separation valve incorporating use of an expandable pH sensor for opening of the valve; Fig. 27a shows the valve in the closed position and Fig. 27b shows the valve in the open position.
Figs. 28a-28b are cross sectional views of an embodiment of a beam like separation valve incorporating use of a contractible pH sensor for opening of the valve; Fig. 28a shows the valve in the closed position and Fig. 28b shows the valve in the open position.
Figs. 29a-29b, show an embodiment of a capsule having tearable seams arranged in a radial or lateral pattern for tearing of the capsule by inflation of the expandable balloon; Fig 29a shows the capsule prior to inflation and Fig. 29b shows the capsule broken into pieces by the inflation of the balloon.
Fig. 30 shows an embodiment of a balloon tearable capsule fabricated from separate portions joined by seams, which can be torn by inflation of the expandable balloon.

### DETAILED DESCRIPTION OF THE INVENTION

Embodiments of the invention provide devices for delivering medications in to various locations in the body. As used herein, the term "medication" refers to a medicinal preparation in any form which can include drugs or other therapeutic agents as well as one or more pharmaceutical excipients. Many embodiments provide a swallowable device for delivering medication within the GI tract. Particular embodiments provide a swallowable device such as a capsule for delivering medications to the wall of the small intestine or other GI organ.

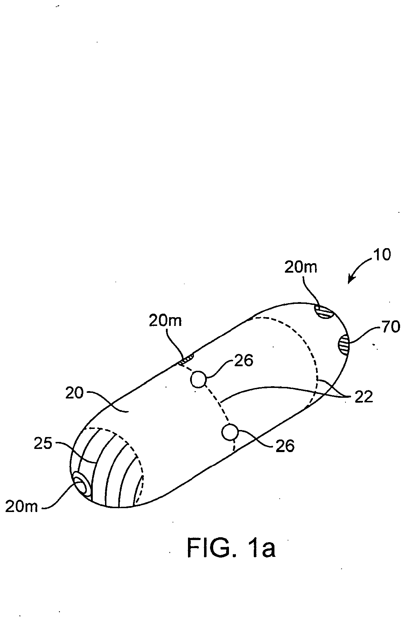

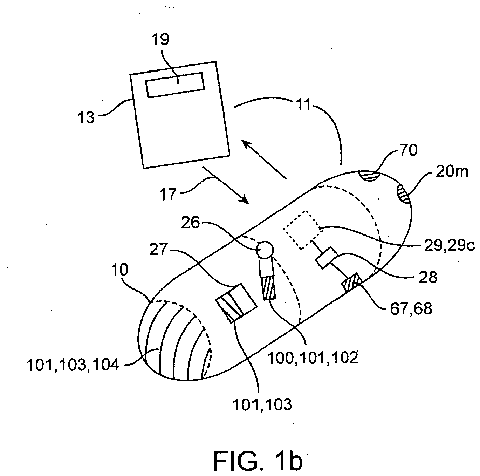

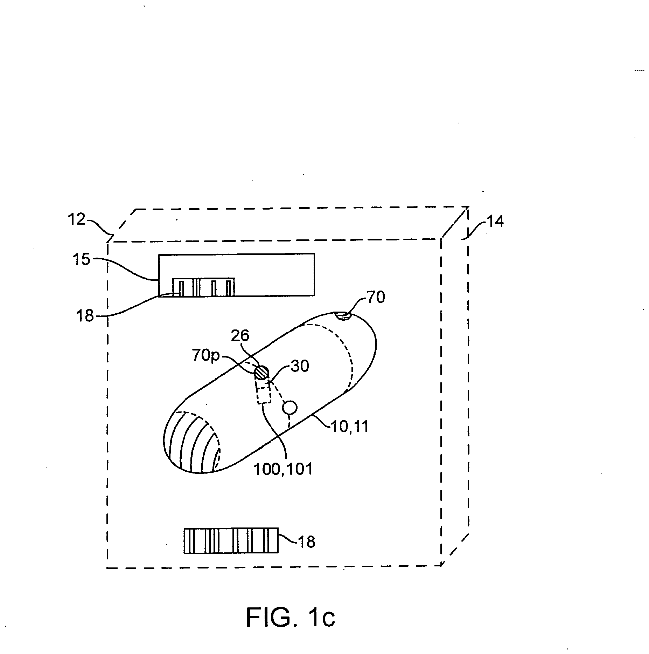

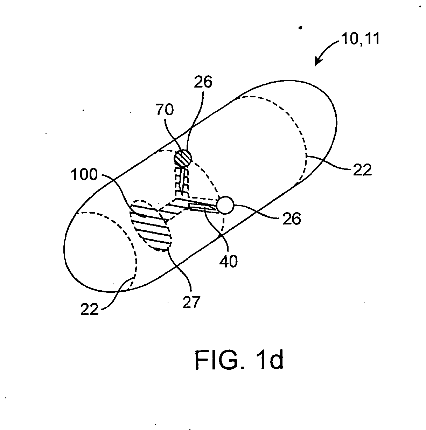

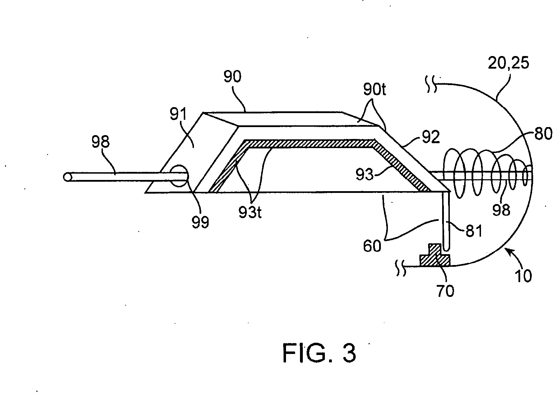

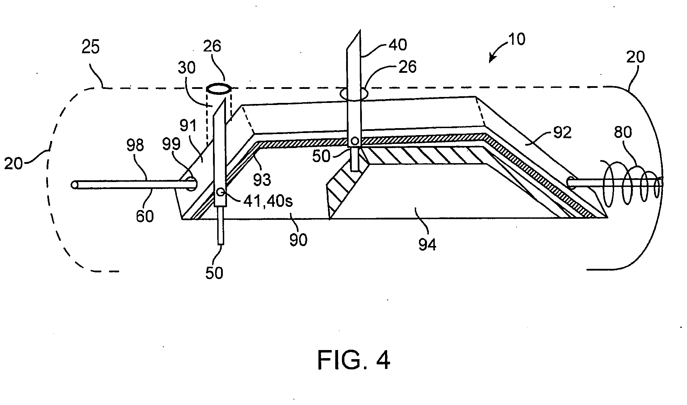

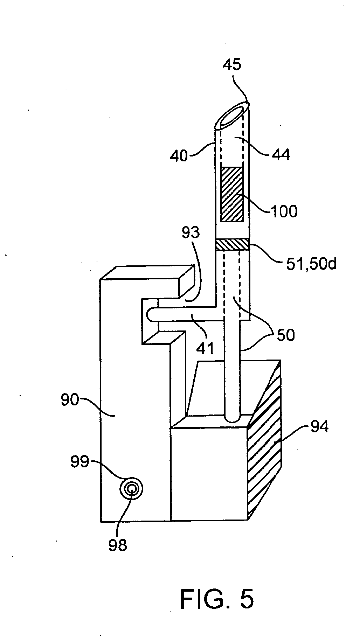

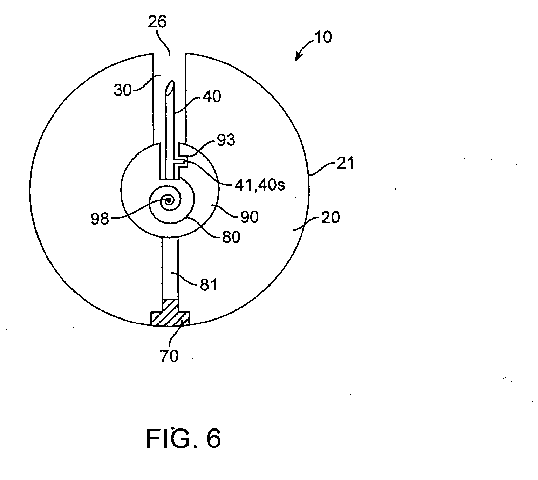

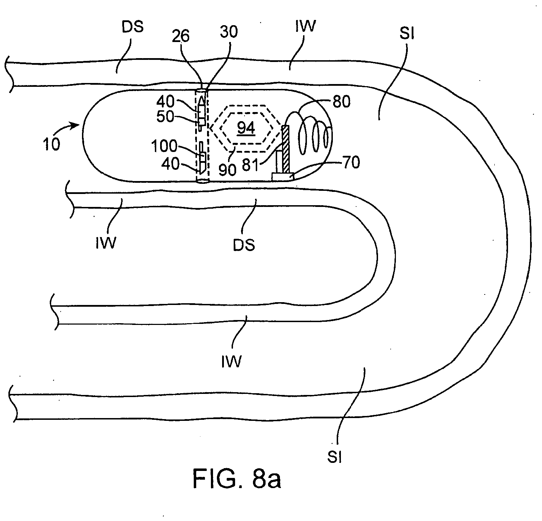

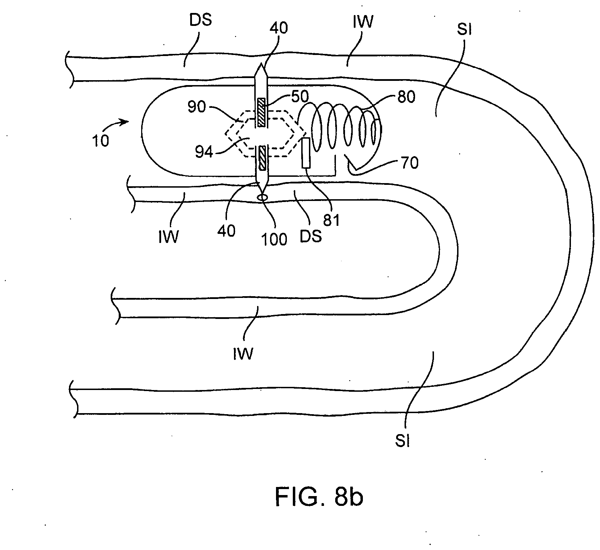

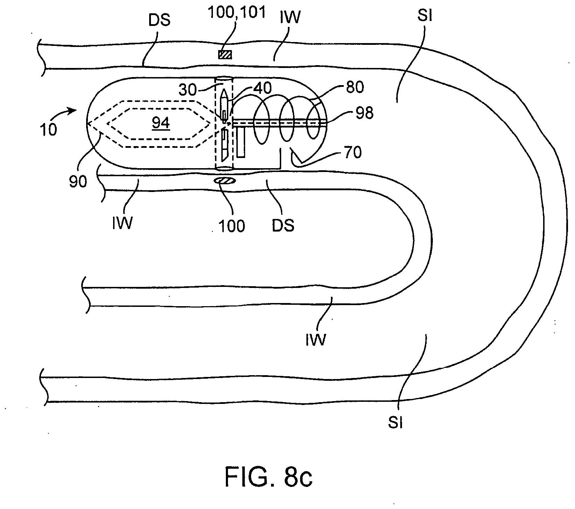

Referring now to Figs. 1-11, embodiments of a device 10 for the delivery of medication 100 to a delivery site DS in the intestinal tract, comprises a capsule 20 including at least one aperture 26, an expandable member 30, guide tube 30, and one or more tissue penetrating members 40 containing a medication 100. The tissue penetrating member 40 can be formed at least in part from medication 100, and/or contain a section or compartment 42 formed from or containing medication 100 that is integral with the tissue penetrating member 40 positioned or otherwise advanceable in the at least one guide tube, a delivery member 50, an actuating mechanism 60 and release element 70. Medication 100 also described herein as preparation 100, typically comprises at least one drug or therapeutic agent 101 and may include one or more pharmaceutical excipients known in the art.

Device 10 including tissue penetrating member 40 can be configured for the delivery of liquid, semi-liquid or solid forms of medication 100 or all three. Solid forms of medication/preparation 100 can include both powder or pellet. Semi liquid can include a slurry or paste. Whatever the form, medication/preparation 100 desirably has a shape and material consistency allowing the medication to be advanced out of the device, into the intestinal wall (or other luminal wall in the GI tract) and then degrade in the intestinal wall to release the drug or other therapeutic agent 101. The material consistency can include one or more of the hardness, porosity and solubility of the preparation (in body fluids). The material consistency can be achieved by one or more of the following: i) the compaction force used to make the preparation; ii) the use of one or more pharmaceutical disintegrants known in the art; iii) use of other pharmaceutical excipients; iv) the particle size and distribution of the preparation (e.g., micronized particles); and v) use of micronizing and other particle formation methods known in the art. Suitable shapes for preparation 100 can include cylindrical, cubical, rectangular, conical, spherical, hemispherical and combinations thereof. Also, the shape can be selected so as to define a particular surface area and volume of preparation 100 and thus, the ratio between the two. The ratio of surface area to volume can in turn, be used to achieve a selected rate of degradation within the intestinal or other lumen wall. Larger ratios (e.g., larger amounts of surface area per unit volume) can be used to achieve faster rates of degradation and vice versa. In particular embodiments, the surface area to volume ratio can be in the range of about 1:1 to 100:1, with specific embodiments of 2:1, 5:1, 20:1, 25:1, 50:1 and 75:1. Medication/preparation 100 will typically be pre-packed within a lumen 44 of tissue penetrating members 40, but can also be contained at another location within an interior 24 of capsule 20, or in the case of a liquid or semi-liquid, within an enclosed reservoir 27. The medication can be pre-shaped to fit into the lumen or packed for example, in a powder form. Typically, the device 10 will be configured to deliver a single drug 101 as part of medication 100. However in some embodiments, the device 10 can be configured for delivery of multiple drugs 101 including a first second, or a third drug which can be compounded into a single or multiple medications 100. For embodiments having multiple medications/drugs, the medications can be contained in separate tissue penetrating members 40 or within separate compartments or reservoirs 27 within capsule 20. In another embodiment, a first dose 102 of medication 100 containing a first drug 101 can be packed into the penetrating member(s) 40 and a second dose 103 of medication 100 (containing the same or a different drug 101) can be coated onto the surface 25 of capsule as is shown in the embodiment of Fig. 1a. The drugs 101 in the two doses of medication 102 and 103 can be the same or different. In this way, a bimodal pharmacokinetic release of the same or different drugs can be achieved. The second dose 103 of medication 100 can have an enteric coating 104 to ensure that it is released in the small intestine and achieve a time release of the medication 100 as well. Enteric coating 104 can include one or more enteric coatings described herein or known in the art.

A system 11 for delivery of medication 100 into the wall of the small intestine or other location within the GI tract, may comprise device 10, containing one or more medications 100 for the treatment of a selected condition or conditions. In some embodiments, the system may include a hand held device 13, described herein for communicating with device 10 as is shown in the embodiment of Fig. 1b. System 11 may also be configured as a kit 14 including system 11 and a set of instructions for use 15 which are packaged in packaging 12 as is shown in the embodiment of Fig. 1c. The instructions can indicate to the patient when to take the device 10 relative to one or more events such as the ingestion of a meal or a physiological measurement such as blood glucose, cholesterol, etc. In such examples , kit 14 can include multiple devices 10 containing a regimen of medications 100 for a selected period of administration, e.g., a day, week, or multiple weeks depending upon the condition to be treated.

Capsule 20 is sized to be swallowed and pass through the intestinal tract. The size can also be adjusted depending upon the amount of drug to be delivered as well as the patient's weight and adult vs. pediatric applications. Typically the capsule will have a tubular shape with curved ends similar to a vitamin. In these and related examples , capsule lengths 20L can be in the range of 12.7 mm to 50.8 mm (0.5 to 2 inches) and diameters 20D in the range of 2.54 mm to 12.7 mm (0.1 to 0.5 inches) with other dimensions contemplated. The capsule 20 includes a capsule wall 21w, having an exterior surface 25 and an interior surface 24 defining an interior space or volume 24v. The capsule wall 21w includes volume 24 and an outer surface 25 having one or more apertures 26 sized for the outward advancement of tissue penetrating members 40. via guide tubes 30. In addition to the other components of device 10, (e.g., the expandable member, actuation mechanism etc.) the interior volume can include one or more compartments or reservoirs 27.

One or more portions of capsule 20 can be fabricated from various biocompatible polymers known in the art, including various biodegradable polymers which in a preferred example can comprise PGLA (polylactic-co-glycolic acid). Other suitable biodegradable materials include various enteric materials described herein as well as lactide, glycolide, lactic acid, glycolic acid, para-dioxanone, caprolactone, trimethylene carbonate, caprolactone, blends and copolymers thereof.

Use of biodegradable materials for capsule 20, including biodegradable enteric materials allows the capsule to degrade in whole or part to facilitate passage through the GI system after drug deliver. As is described in further detail herein, in various examples, capsule 20 can include seams 22 of bio-degradable material so as to controllably degrade into smaller pieces 23 which are more easily passed through the intestinal tract.

Additionally, in various examples , the capsule 20 can include various radio-opaque or echogenic materials for location of the device using fluoroscopy, ultrasound or other medical imaging modality. In specific examples , all or a portion of the capsule can include radio-opaque/echogenic markers 20m as is shown in the example of Figs 1a and 1b. In use, such materials not only allow for the location of device 10 in the GI tract, but also allow for the determination of transit times of the device through the GI tract.

Expandable member 30 can comprise a variety of expandable devices shaped and sized to fit within capsule 20, but will typically comprise an expandable balloon 30. Other suitable expandable members include various shape memory devices, and/or chemically expandable polymer devices having an expanded shape and size corresponding to the interior volume 24v of the capsule 20. For ease of discussion, expandable member 30 will now be referred to as balloon 30, but other embodiments are equally applicable. Balloon 30 will typically be attached to an interior surface 24 of the capsule 20 in at least a partially non-expanded state. Means of attachment can include the use of various adhesive known in the medical device arts. The balloon can be packed inside capsule 20 in a furled or other compact configuration to conserve space within the interior portion of the capsule. Furling may be achieved by placement of separation valve 50 over a selected portion of the un-inflated balloon 30. In a particular embodiments, furling can be facilitated by the use of a collar type separation valve 55 described herein that is placed around the un-inflated balloon to hold in a furled configuration. In another approach, furling can also be achieved by the use of one or more pre-formed creases 30c placed along the balloon in a lateral, spiral or other configuration. In preferred embodiments, tissue penetrating members 40 are positioned within guide tubes 30 which serve to guide and support the advancement of members 40 into tissue such as the wall of the small intestine or other portion of the GI tract. In other embodiments, tissue penetrating members 40 can be positioned in capsule 20 without guide tubes. The tissue penetrating members 40 will typically comprise a hollow needle or other like structure and will have a lumen 44 and a tissue penetrating end 45 for penetrating a selectable depth into the intestinal wall IW. Member 40 may also include a pin 41 for engagement with a motion converter 90 described herein. The depth of penetration can be controlled by the length of member 40, the configuration of motion converter 90 described herein as well as the placement of a stop or flange 40s on member 40 which can, in an embodiment, correspond to pin 41 described herein. Medication 100 will typically be delivered into tissue through lumen 44. In many embodiments, lumen 44 is pre-packed with the desired medication 100 which is advanced out of the lumen using delivery member 50 or other advancement means (e.g. by means of force applied to a collapsible embodiment of member 40). As an alternative, medication 100 can be advanced into lumen 44 from another location/compartment in capsule 20. In some embodiments, all or a portion of the tissue penetrating member 40 can be fabricated from medication 100 itself. In these and related examples , the medication can have a needle or dart-like structure (with or without barbs) configured to penetrate and be retained in the intestinal wall such as the wall of the small intestine. The dart can be sized and shaped depending upon the medication, dose and desired depth of penetration into the intestinal wall. Medication 100 can be formed into darts, pellets or other shapes using various compression molding and other related methods known in the pharmaceutical arts.

Balloon 30 can comprise various polymers known in the medical device arts, but preferably comprises non-compliant polymers such as PET (Polyethylene Teraphalate) and other non compliant materials known in the art. It can be fabricated using various balloon blowing methods known in the balloon catheters arts (e.g., mold blowing) to have a shape 30s and size which corresponds approximately to the interior volume 24v of capsule 20. Suitable shapes 30s for balloon 30 include various cylindrical shapes having tapered or curved end portions 31 (an example of such a shape including a hot dog). In some examples , the inflated size of balloon 30, including its diameter 30D can be slightly larger than capsule 20 so as to cause the capsule to come apart from the force of inflation, (e.g., due to hoop stress). Desirably, the walls 32 of balloon 30 will be thin and can have a wall thickness 33 in the range of 0.127 mm to 0.00254 mm (0.005 to 0.0001") more preferably, in the range 0.0254 mm to 0.00254 mm (0.001 to 0.0001"), with specific examples of 0.0508, 0.0254 and 0.0127 mm (0.002, 0.001, and 0.0005").

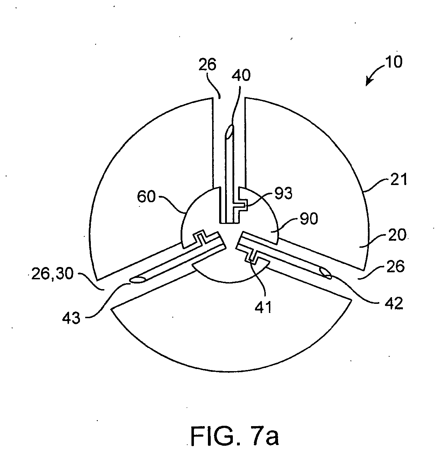

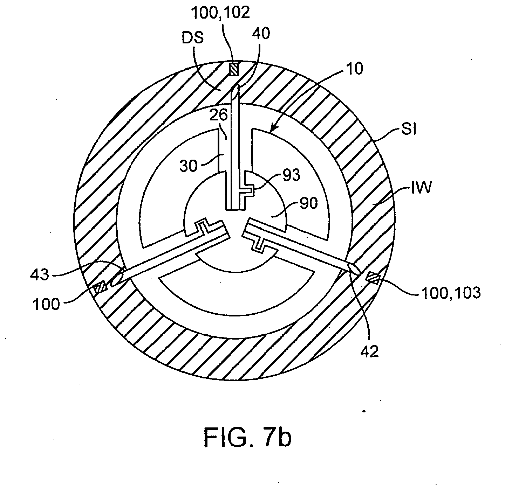

In various examples , device 10 can include a second 42 and a third 43 tissue penetrating member 40 as is shown in the examples of Figs. 7a and 7b., with additional numbers contemplated. Each tissue penetrating member 40 can be used to deliver the same or a different medication 100 as well as different doses of the same drug. In preferred examples , the tissue penetrating members 40 can be substantially symmetrically distributed around the perimeter 21 of capsule 20 so as to anchor the capsule onto the intestinal wall IW during delivery of medications 100. Anchoring capsule 20 in such a way reduces the likelihood that the capsule will be displaced or moved by peristaltic contractions occurring during delivery of the medication. In specific examples , the amount of anchoring force can be adjusted to the typical forces applied during peristaltic contraction of the small intestine. Anchoring can be further facilitated by configured some or all of tissue penetrating members 40 to have a curved or arcuate shape.

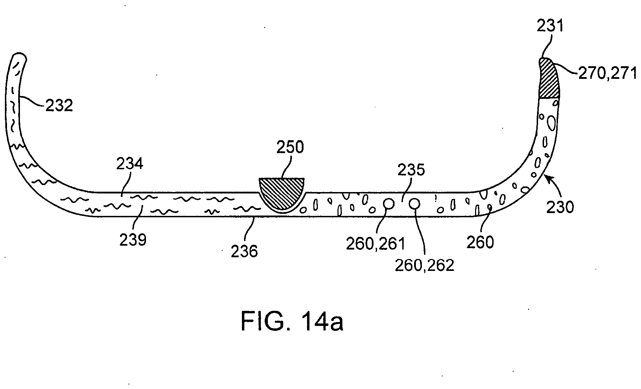

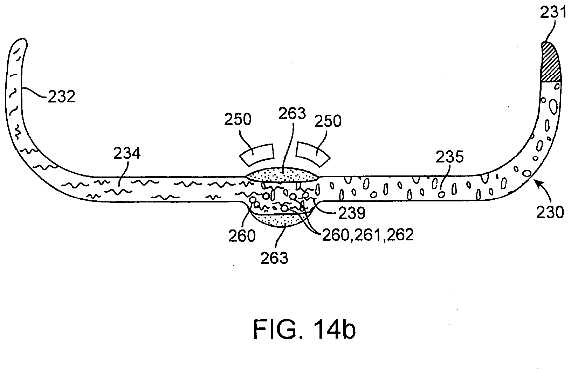

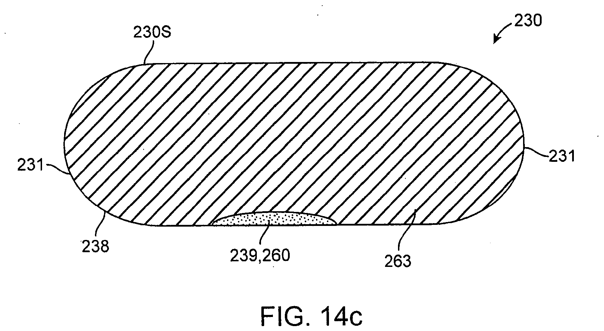

Balloon 230 also will typically include at least a first and a second portion or compartment 234 and 235 which are separated by a separation valve, delivery member, or other separation means which separates the contents of each compartment. In many embodiments, compartments 234 and 235 will have at least a small connecting section 236 between them which is where separation valve 250 will typically be placed. A liquid 239, typically water, can be disposed within first compartment 234 and one or more reactants 260 disposed in second compartment 235 (which typically are solid though liquid may also be used) as is shown in the embodiment of Fig. 14a. When valve 250 opens (e.g., from degradation caused by fluids within the small intestine) liquid 239 enters compartment 235 (or vice versa or both), the reactant(s) 260 mix with the liquid and produce a gas 263 such as carbon dioxide which expands balloon 230 as is shown in the embodiments of Figs. 14b-14c. Expansion of balloon 230 is configured to advance medication 100 through the tissue penetrating member 240 into the intestinal wall IW as will be explained more fully herein. Accordingly, at least a portion of the delivery member 250 is advanceable within the tissue penetrating member lumen 244 and thus member 250 has a size and shape (e.g., a piston like shape) configured to fit within the delivery member lumen 244 or other chamber or compartment within tissue penetrating member 240.

Reactants 260 will typically include at least a first and a second reactant, 261 and 262 for example, an acid such as citric acid and a base such as sodium hydroxide. Additional numbers of reactants are also contemplated. For embodiments using citric acid and sodium hydroxide, the ratio's between the two reactants (citric acid to sodium hydroxide) can be in the range of 1:1 to 1:4, with a specific ratio of 1:2. Desirably, solid reactants 260 have little or no absorbed water. Accordingly, one or more of the reactants, such as sodium hydroxide can be pre-dried (e.g., by vacuum drying) before being placed within balloon 230. Other reactants 260 including other acids, e.g., ascetic acid and bases are also contemplated. The amounts of particular reactants 260, including combinations of reactants can be selected to produce particular pressures using known stoichiometric equations for the particular chemical reactions as well as the inflated volume of the balloon and the ideal gas law (e.g., PV=nRT)

In some embodiments, the distal end 50d of the delivery member (the end which is advanced into tissue) can have a plunger element 51 which advances the medication within the tissue penetrating member lumen 44 and also forms a seal with the lumen. Plunger element 51 can be integral or attached to delivery member 50. Preferably, delivery member 50 is configured to travel a fixed distance within the needle lumen 44 so as to deliver a fixed or metered dose of drug into the intestinal wall IW. This can be achieved by one or more of the selection of the diameter of the delivery member (e.g., the diameter can be distally tapered), the diameter of the tissue penetrating member (which can be narrowed at its distal end), use of a stop, and/or the actuating mechanism. However in some embodiments, the stroke or travel distance of member 50 can be adjusted in situ responsive to various factors such as one or more sensed conditions in the GI tract. In situ adjustment can be achieved through use of logic resource 29 (including controller 29c) coupled to an electro-mechanical embodiment of actuating mechanism 60. This allows for a variable dose of medication and/or variation of the distance the medication is injected into the intestinal wall.

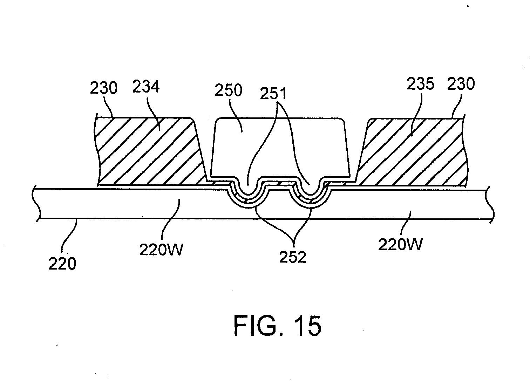

Various embodiments of the invention provide a number of structures and configurations for a separation valve 250 or other separation means 250. As is described below, in one or more embodiments, valve 250 may comprise a beam like structure, or collar type valve. Still other structures are considered. In one or more of these embodiments, valve 250 can include one or more pinching features 251 such as a ridge which engages a depression or other mating feature 252 on the internal surface 224 of capsule 220 as is shown in the embodiment of Fig. 15. In use, pinching features 251 provide for the application of additional force on the balloon wall 232 beneath the pinching feature and redundancy to the seal. Valve 250 may include multiple pinching features 251 to create a seal under each feature.

Actuating mechanism 60 can be coupled to at least one of the tissue penetrating member 40 or delivery member 50. The actuating mechanism is configured to advance tissue penetrating member 40 a selectable distance into the intestinal wall IW as well as advance the delivery member to deliver medication 100 and then withdraw the tissue penetrating member from the intestinal wall. In various embodiments, actuating mechanism 60 can comprise a spring loaded mechanism which is configured to be released by release element 70. Suitable springs 80 can include both coil (including conical shaped springs) and leaf springs with other spring structures also contemplated. In particular embodiments, spring 80 can be substantially cone-shaped to reduce the length of the spring in the compressed state even to the point where the compressed length of the spring is about the thickness of several coils (e.g., two or three) or only one coil.

Also in various embodiments, separation valve 250 can be configured to open in a number of ways and responsive to a number of conditions within the GI tract. In many embodiments, the separation valve 250 will be configured to open by having one or more portions degrade in response to the higher pH or other conditions found within the small intestine such that upon degradation, the valve opens. As an alternative or additional approach, separation valve 250 may also be configured to open in response to compressive forces applied by a peristaltic contraction within the small intestine. In still another approach, separation valve 250 may be a time-release valve configured to open after a certain period of time after a trigger event, e.g., an activation step initiated by the patient such as the pealing of a tab or pressing of a button.

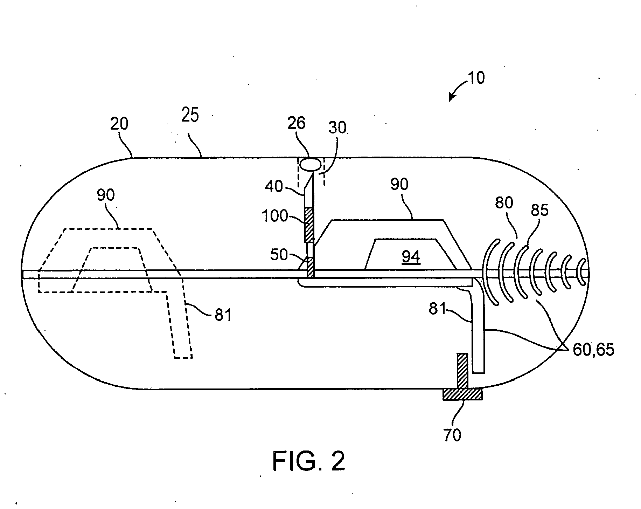

In particular embodiments actuating mechanism 60 can comprise a spring 80, a first motion converter 90, and a second motion converter 94 and a track member 98 as is shown in the embodiments of Figs. 2, 4 and 8a-8c. The release element 70 is coupled to spring 80 to retain the spring in a compressed state such that degradation of the release element releases the spring. Spring 80 may be coupled to release element 70 by a latch or other connecting element 81. First motion converter 90 is configured to convert motion of spring 80 to advance and withdraw the tissue penetrating member 40 in and out of the intestinal wall or other tissue. The second motion converter 94 is configured to convert motion of the spring 80 to advance the delivery member 50 into the tissue penetrating member lumen 44. Motion converters 90 and 94 are pushed by the spring and ride along a rod or other track member 98 which fits into a track member lumen 99 of converter 90. The track member 98 which serves to guide the path of the converters 90. Converters 90 and 94 engage the tissue penetrating member 40 and/or delivery member 50 (directly or indirectly) to produce the desired motion. They have a shape and other characteristics configured to convert motion of the spring 80 along its longitudinal axis into orthogonal motion of the tissue penetrating member 40 and/or delivery member 50 though conversion in other directions is also contemplated. The motion converters can have a wedge, trapezoidal or curved shape with other shapes also contemplated. In particular embodiments, the first motion converter 90 can have a trapezoidal shape 90t and include a slot 93 which engages a pin 41 on the tissue penetrating member that rides in the slot as is shown in the embodiments of Figs. 2, 3 and 4. Slot 93 can also have a trapezoidal shape 93t that mirrors or otherwise corresponds to the overall shape of converter 90. Slot 93 serves to push the tissue penetrating member 40 during the upslope portion 91 of the trapezoid and then pull it back during the down slope portion 92. In one variation, one or both of the motion converters 90 and 94 can comprise a cam or cam like device (not shown). The cam can be turned by spring 80 so as to engage the tissue penetrating and/or delivery members 40 and 50. One or more components of mechanism 60 (as well as other components of device 10) including motion converters 90 and 94 can be fabricated using various MEMS-based methods known in the art so as to allow for selected amounts of miniaturization to fit within capsule 10. Also as is described herein, they can be formed from various biodegradable materials known in the art.

Examples of a degradable separation valve 250 can be positioned in a variety of locations on or within capsule 220 so as to exposed to and degraded by the intestinal fluids. While at least a portion of the valve may be exposed to the capsule exterior surface 225, typically, the valve will be positioned within the capsule interior 224v where it is exposed to intestinal fluids which enter through the at least one aperture 226 or other opening. In these and related examples , at least a portion of the capsule exterior surface 225 including the portion containing the at least one aperture 226 is desirably coated with a protective layer or coating 220c, such as an enteric coating which also degrades in response to pH or other conditions within the small intestine. Typically, the entire capsule will be so coated, however in some embodiments only a portion over apertures 226 will be coated. Such coatings provide a protective seal 226s over the at least one aperture 226 so that digestive fluids do not enter the capsule interior 224v and start to degrade the separation valve 250 until the capsule has reached the small intestine. The examples of Figs. 16a-16c illustrate the sequence of degradation of the coating, ingress of intestinal or other fluid F into the capsule interior and subsequent degradation of the separation valve. In use, examples of device 210 employing a degradable coating 220c over the aperture 226 and a degradable valve 250 provide a primary and secondary seal for assuring that balloon 230 does not prematurely expand and deploy its tissue penetrating members 240 until capsule 220 has reached the small intestine.

In other variations, the actuating mechanism 60 can also comprise an electro-mechanical device/mechanism such as a solenoid, or a piezoelectric device. In one examples, a piezoelectric device used in mechanism 60 can comprise a shaped piezoelectric element which has a non-deployed and deployed state. This element can be configured to go into the deployed state upon the application of a voltage and then return to the non-deployed state upon the removal of the voltage. This and related examples allow for a reciprocating motion of the actuating mechanism 60 so as to both advance the tissue penetrating member and then withdraw it. The voltage for the piezoelectric element can be obtained generated using a battery or a piezoelectric based energy converter which generates voltage by mechanical deformation such as that which occurs from compression of the capsule 20 by a peristaltic contraction of the small intestine around the capsule. Further description of piezoelectric based energy converters is found in U.S Patent Application Serial No. 12/556,524.

In one example , deployment of tissue penetrating members 40 can in fact be triggered from a peristaltic contraction of the small intestine which provides the mechanical energy for generating voltage for the piezoelectric element.

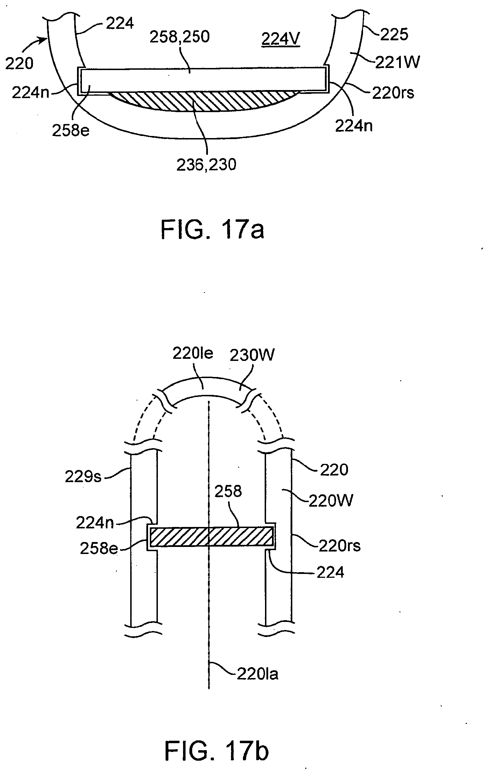

According to one or more examples , separation valve 250 may comprise a beam-like structure 258 that is placed within capsule 220 to compress and seal the portion of the balloon 236 between the first and second compartments 234 and 235 as is shown in the example of Figs. 17a and 17b. Beam 258 is desirably constructed of one more degradable materials described herein, e.g., PGLA, cellulose, etc. which degrade in response to the fluids found within the small intestine. When beam 258 degrades, the compressive forces of the balloon are released and contents from the first and second compartments 234 and 235 mix causing balloon expansion as described herein. Beam 258 can be attached at one or both sides of the interior surface 224 of the capsule. Typically, the beam will be placed proximate a central portion 236 of balloon 230, though other locations are also contemplated. In preferred examples , the beam 258 is positioned in radially oriented fashion with respect to balloon lateral axis 20la, attached to the radial sides 220rs of capsule interior surface 224 as is shown in the example of Figs. 17a and 17b. However, beam 258 may also be attached to the lateral ends 20le of the capsule interior surface. Preferably, in either of these two examples , beam 258 is attached to capsule interior surface 224 using an interference fit so that the beam can be snapped into place within the capsule using pick and place and other like methods known in the manufacturing arts. In specific examples , interior surface 224 can include notches 224n for placement of beam ends 258e to allow a snap or press fit of the beam 258 into the capsule 220.

Release element 70 will typically be coupled to the actuating mechanism 60 and/or a spring coupled to the actuating mechanism; however other configurations are also contemplated. In preferred embodiments, release element 70 is coupled to a spring 80 positioned within capsule 20 so as to retain the spring in a compressed state 85 as shown in the embodiment of Fig. 2. Degradation of the release element 70 releases spring 80 to actuate actuation mechanism 60. Accordingly, release element 70 can thus function as an actuator 70a (actuator 70a may also include (singularly or coupled to release element 70) spring 80 and other elements of mechanism 60). As is explained further below, actuator 70a has a first configuration where the therapeutic agent preparation 100 is contained within capsule 20 and a second configuration where the therapeutic agent preparation is advanced from the capsule into the wall of the small intestine or other luminal wall in the intestinal tract.

In many embodiments, release element 70 comprises a material configured to degrade upon exposure to chemical conditions in the small or large intestine such as pH. Typically, release element 70 is configured to degrade upon exposure to a selected pH in the small intestine, e.g., 7.0, 7.1, 7.2, 7.3, 7.4, 7.5, 7.6 8.0 or greater. The release element can also be configured to degrade within a particular range of pH such as, e.g., 7.0 to 7.5. In particular embodiments, the pH at which release element 70 degrades (defined herein as the degradation pH) can be selected for the particular drug to be delivered so as to release the drug at a location in small intestine which corresponds to the selected pH. Further, for embodiments of device 10 having multiple medications 100, the device can include a first release element 70 (coupled to an actuating mechanism for delivering a first drug) configured to degrade at first pH and a second release element 70 (coupled to an actuating mechanism for delivering a second drug) configured to degrade at a second pH (with additional numbers of release elements contemplated for varying number of drugs).

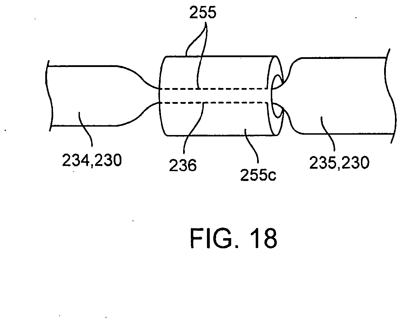

According to another embodiment shown in Fig. 18, the separation valve 250 can comprise a collar valve 255 including a connecting 236 of the expandable member 230 with an overlying constricting collar 255c made from biodegradable material. Collar 255c holds connection section 236 closed and releases it when the collar is degraded.

Release element 70 can also be configured to degrade in response to other conditions in the small intestine (or other GI location). In particular embodiments, the release element 70 can be configured to degrade in response to particular chemical conditions in the fluids in the small intestine such as those which occur after ingestion of a meal (e.g., a meal containing fats, starches or proteins). In this way, the release of medication 100 can be substantially synchronized or otherwise timed with the digestion of a meal. Such embodiments are particularly useful for the delivery of medication to control levels of blood sugar/glucose (e.g., insulin), serum cholesterol and serum triglycerides.

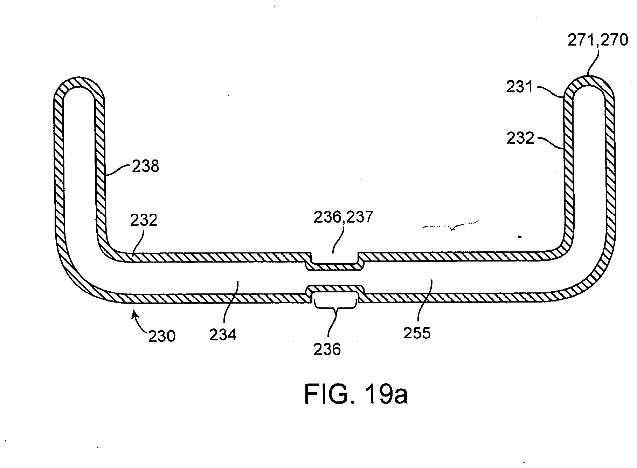

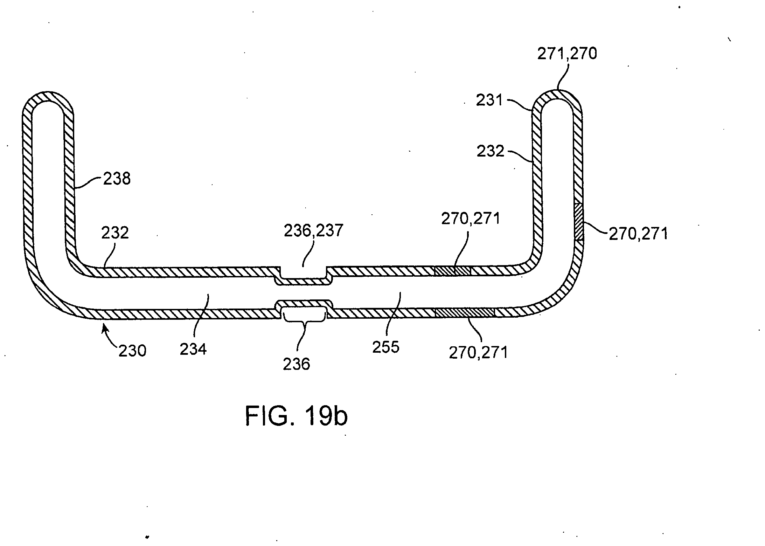

In addition to release valve 250, the balloon or other expandable member 230 will also typically include a deflation valve 270 which serves to deflate balloon 230 after inflation. Deflation valve 270 can comprise biodegradable materials which are configured to degrade upon exposure to the fluids in the small intestine and/or liquid in one of the compartments of the balloon so as to create an opening or channel for escape of gas within balloon. In one embodiment shown in Fig. 19a, the deflation valve 270 can comprise a biodegradable section 271 positioned on an end portion 231 of the balloon 230 so as to join opposing ends of the balloon wall 232 together. In this and related embodiments, when degradable section 271 degrades from exposure to the liquid, balloon wall 232 tears or otherwise comes apart providing for a high assurance of rapid deflation. Multiple degradable sections 271 can be placed at various locations within balloon wall 232 is shown in the embodiment of Fig. 19b, to provide an even higher degree of reliability in deflation. Desirably, sections 271 are only placed within the wall 232 of compartment 235. For embodiments where the deflation valve 270 is degraded by fluids within the small intestine, degradation of the valve can be facilitated by configuring inflated balloon 230 to break apart capsule 220 into two or more pieces so that large sections of the balloon are directly exposed to degrading fluids within the small intestine. This can be achieved by fabricating capsule 220 from separate parts (e.g., two halves mechanically fit together) and/or through the use of seams 222 in the capsule wall as is described herein.

Various approaches are contemplated for biodegradation of release element 70. In particular embodiments, biodegradation of release element 70 from one or more conditions in the small intestine (or other location in the GI tract) can be achieved by one or more of the following: i) selection of the materials for the release element, ii) the amount of cross linking of those materials; and iii) the thickness and other dimensions of the release element. Lesser amounts of cross linking and or thinner dimensions can increase the rate of degradation and visa versa. Suitable materials for the release element can comprise biodegradable materials such as various enteric materials which are configured to degrade upon exposure to the higher pH in the intestines. Suitable enteric materials include, but are not limited to, the following: cellulose acetate phthalate, cellulose acetate trimellitate, hydroxypropyl methylcellulose phthalate, polyvinyl acetate phthalate, carboxymethylethylcellulose, co-polymerized methacrylic acid/methacrylic acid methyl esters as well as other enteric materials known in the art. The selected enteric materials can be copolymerized or otherwise combined with one or more other polymers to obtain a number of other particular material properties in addition to biodegradation. Such properties can include without limitation stiffness, strength, flexibility and hardness.

Additionally, as further backup for insured deflation, one or more puncture elements 72 can be attached to the inside surface 24 of the capsule wall such that when the balloon fully deflates it is contacts and is punctured by the puncture element. Puncture elements 72 can comprise short protrusions from surface 24 having a pointed tip 73. In another alternative or additional embodiment of means for balloon deflation, one or more of the tissue penetrating members 40 can be directly coupled to balloon wall 32 and configured to tear away from the balloon when they detach, tearing the balloon wall in the process.

In alternative embodiments, the release element 70 can comprise a film or plug 70p that fits over or otherwise blocks guide tubes 30 and retains the tissue penetrating member 40 inside the guide tube (Fig. 1c). In these and related embodiments, tissue penetrating member 40 is coupled to a spring loaded actuating mechanism such that when the release element is degraded sufficiently, it releases the tissue penetrating member which then springs out of the guide tube to penetrate into the intestinal wall. In still other embodiments, release element 70 can be shaped to function as a latch which holds the tissue penetrating member 40 in place. In these and related embodiments, the release element can be located on the exterior or the interior of capsule 20. In the latter case, capsule 20 and/or guide tubes 30 can be configured to allow for the ingress of intestinal fluids into the capsule interior to allow for the degradation of the release element.

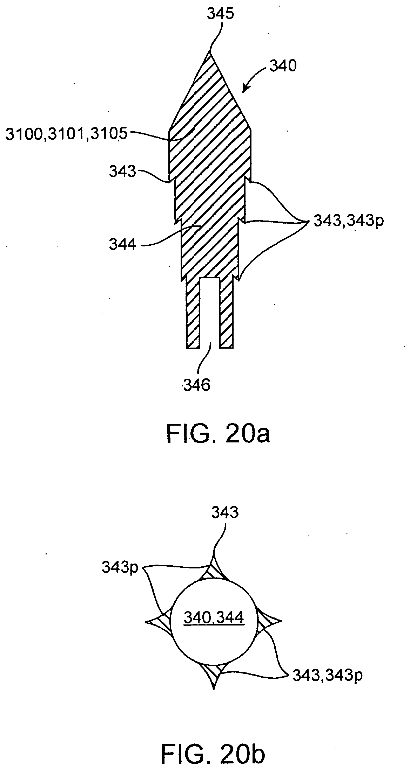

Tissue penetrating member 40 can be fabricated from various drugs and other therapeutic agents 101 as well as one or more biodegradable polymers to provide desired structural properties to the penetrating member (e.g., column strength) and/or control the release of drug. Referring now to Figs. 20a-20c, in many embodiments, the penetrating member 40 can be formed to have a shaft 44 and a needle tip 45 or other pointed tip 45 so as to readily penetrate tissue of the intestinal wall as shown in the embodiment of Fig. 20a. Tip 45 may comprise degradable materials (within the body of the tip or as a coating), such as sucrose which increase the hardness and tissue penetrating properties of the tip. Once placed in the intestinal wall, the penetrating member 40 is degraded by the interstitial fluids within the wall tissue, the drug dissolves in those fluids and is absorbed into the blood stream. Penetrating member 40 will also typically include one or more tissue retaining features 43 such as a barb or hook to retain the penetrating member within the tissue of the intestinal wall after advancement. Retaining remembers 43 can be arranged in various patterns 43p to enhance tissue retention such as two or more barbs symmetrically or otherwise distributed around and along member shaft 44 as is shown in the embodiments of Figs. 20a and 20b. Additionally, in many embodiments, penetrating member will also include a recess or other mating feature 46 for attachment to a coupling component which attaches the penetrating member to the balloon (such as advancement member 80a described below).

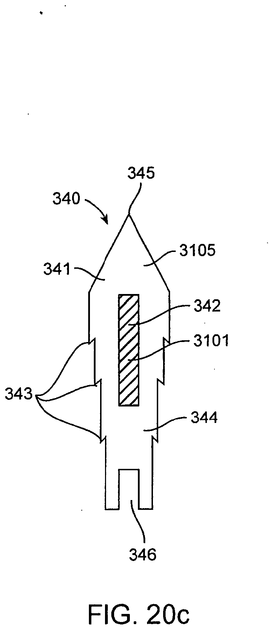

As described above, tissue penetrating member 340 can be fabricated from a number of drugs and other therapeutic agents 3101. The penetrating member may be fabricated entirely from drug 3101 or may have other constituent components as well, e.g., various pharmaceutical excipients. Typically, the drug or other therapeutic agent 3101 will be mixed in with a biodegradable polymer 3105 such as PGLA, cellulose or other biodegradable material described herein or known in the art. In such embodiments, the penetrating member 340 may comprise a substantially heterogeneous mixture of drug 3101 and biodegradable polymer 3105. Alternatively, the penetrating member may 340 include a 341 portion formed substantially from biodegradable material 3105 and a separate section or compartment 342 that is formed from or contains drug 3101 as shown in the embodiment of Fig. 20c.

Tissue penetrating member 340 can be fabricated using one or more polymer and pharmaceutical fabrication techniques known in the art. For example, drug 3101 (with or without biodegradable material 3105) can be in solid form and then formed into the shape of the tissue penetrating member 340 using molding, compaction or other like method with one or more binding agents added. Alternatively, drug 3101 and/or drug preparation 3100 may be in solid or liquid form and then added to the biodegradable polymer 3105 in liquid form with the mixture then formed into the penetrating member 340 using molding or other forming method known in the polymer arts.

Desirably, embodiments of the tissue penetrating member 340 comprising a drug or other therapeutic agent 3101 and degradable polymer 3105 are formed at temperatures which do not produce any substantial thermal degradation of drug including drugs such as various peptides and proteins. This can be achieved through the use of room-temperature curing polymers and room temperature molding and solvent evaporation techniques known in the art. In particular embodiments, the amount of thermally degraded drug or other therapeutic agent within the tissue penetrating member is desirably less than about 10% by weight and more preferably, less than 5% and still more preferably less than 1%. The thermal degradation temperature(s) for a particular drug are either known or can be determined using methods known in the art and then this temperature can be used to select and adjust the particular polymer processing methods (e.g., molding, curing. solvent evaporation methods etc.) to minimize the temperatures and associated level of drug thermal degradation.

Tissue penetrating member 340 is desirably configured to be detachably coupled (directly or indirectly) to the balloon or other expandable member 330 so that after advancement of the tissue penetrating member 340 into the intestinal wall, the penetrating member detaches from the balloon. Detachability can be implemented by a variety of means including: i) the configuration and strength of the joint between penetrating member 340 and advancement member 380a (or other intermediary component(s) coupling member 340 to balloon 330); 2) the configuration and placement of tissue retaining features 343 on penetrating member 340; and iii) the depth of penetration of shaft 344 into the intestinal wall. Using one or more of these factors, penetrating member 340 be configured to detach as a result of balloon deflation (where the retaining features 343 hold the penetrating member in tissue as the balloon deflates or otherwise pulls back away from the intestinal wall) and/or the forces exerted on capsule 320 by a peristaltic contraction of the small intestine.

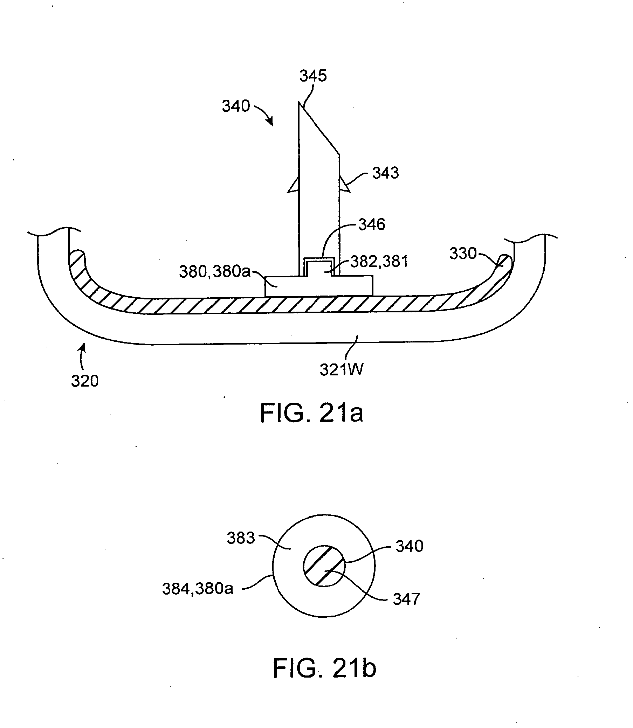

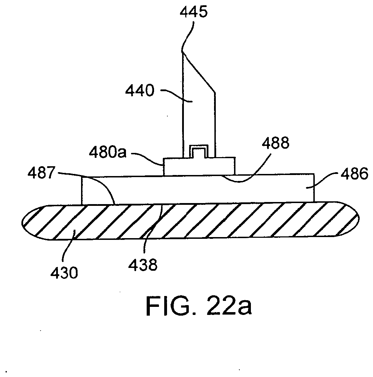

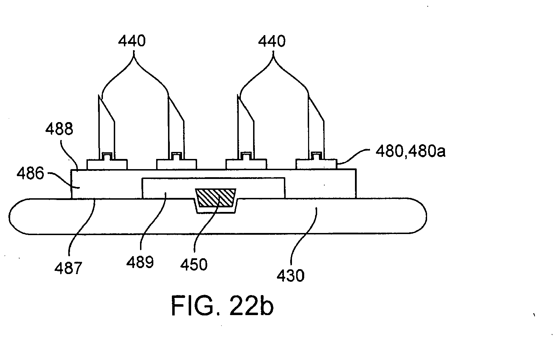

Tissue penetrating member 340 can be directly or indirectly coupled to balloon 330. Referring now to Figs 21a-21b and 22, indirect coupling can be implemented using one or more coupling components 380 such as an advancement member 380a. Accordingly, in particular embodiments, the tissue penetrating member 340 may be coupled to balloon 330 by an advancement member 380a comprising a rigid structure attached to the balloon surface 338 which detachably engages the penetrating member 340. The advancement member 380a engages the penetrating member 340 by means of an attachment feature 381 such as a pin or other protrusion 382 (integral or attached to member 380a) which fits into a recess or other mating feature 346 of the penetrating member as is shown in the embodiment of Fig. 21a. The pin 382 and recess 346 can be configured to detach from the force of balloon deflation and/or force applied to capsule 320 by peristaltic contraction. In many embodiment, advancement member 380a can have a larger horizontal surface area 383 than the surface area 347 of penetrating member 340 so as to function as a force concentration element 384 as is shown in the embodiment of Fig 21b. In use force concentration element 384 functions to increase the force per unit area applied to the penetrating member from expansion of balloon 330 or other expandable member.

In some embodiments, the advancement member 480a can be coupled to the balloon 430 via a support member 486 as is shown in the embodiments of Figs. 22a and 22b. Support member 486 may correspond to a platform 486 having one surface 487 attached to the balloon surface 438 and the other surface 488 attached to the advancement member 480a (one or both of these attachments can be an adhesive attachment) as is shown in the embodiment of Fig. 22a. Platform 486 is desirably rigid, can have a plate-like structure and can be sized to allow for attachment and advancement of multiple advancement members 480a and tissue penetrating members 440 at the same time as is shown in the embodiment of Fig 22b. For example, in particular embodiments, three, four or five groups of advancement and tissue penetrating members can be attached to platform 486, with additional numbers contemplated. In such embodiments, the platform may include a recess 489 for positioning of isolation valve 450.

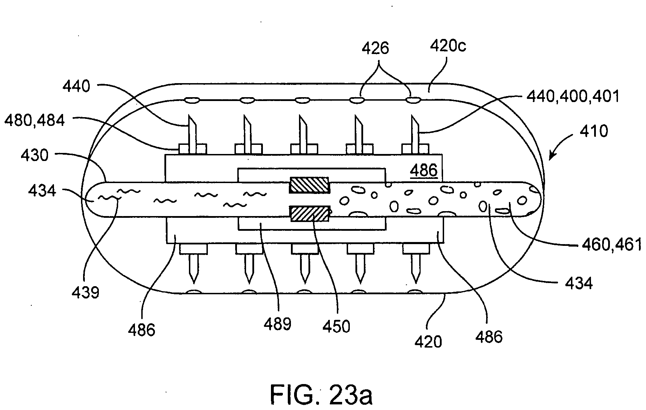

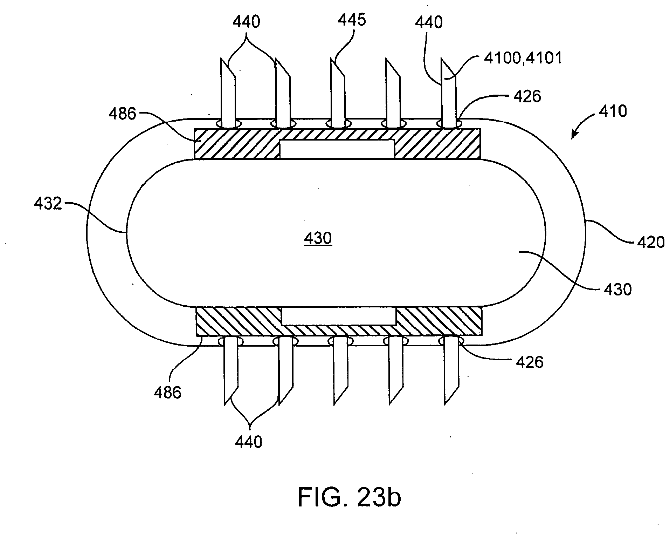

Also, platforms 486 can be placed on either side of balloon 430 to allow for bilateral deployment of tissue penetrating members 440 into intestinal wall IW as is shown in the embodiment of Figs 23a and 23b. In addition to delivering more drug, bilateral deployment serves to anchor capsule 420 on both sides of the intestinal wall IW during deployment of penetrating members 440, thus reducing the likelihood of the capsule from being dislodged during deployment (e.g., due to peristaltic contraction). In these and related embodiments tissue penetrating members 440 can be directly coupled to platform 486 without necessarily using advancement members 480a. Desirably, both advancement members 480 and platform 486 are constructed from biodegradable materials such as PGLA, which can be cross linked and/or copolymerized with to have increased rigidity to support the advancement of penetrating members 440 into tissue.

As an additional or alternative embodiment to the use of advancement member 480a and/or platform 468, tissue penetrating members 440 may be directly coupled to the balloon 430, e.g., by an adhesive where the adhesive force is less than the necessary to pull penetrating member out of tissue once it is deployed into the intestinal wall. In these and related embodiments, the tissue penetrating members 440 may also be configured to tear the balloon wall 432 when they detach from the balloon and thus provide a means for balloon deflation.

In various embodiments, penetrating members 440 can carry the same or a different drug 4101 or other therapeutic agent. The former configuration allows for the delivery of greater amounts of a particular drug 4101, while the later, allows two or more different drugs to be delivered into the intestinal wall at about the same time to facilitate drug treatment regimens requiring substantial concurrent delivery of multiple drugs.

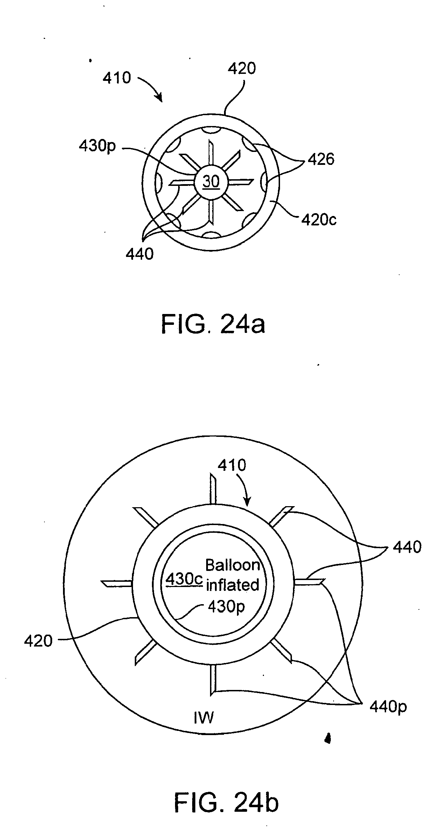

In various embodiments, depending upon the drug and associated drug regimen (e.g., dose and times per day, etc), tissue penetrating members 440 can be placed and distributed in a number of locations and patterns on the balloon surface. As described above for the embodiments of Fig. 23a and 23b, tissue penetrating members 440 can be placed on opposite sides of balloon surface 438 so that balloon inflation can place tissue penetrating members 440 on opposite sides of the intestinal wall IW. Referring now to Figs. 24a-24b, in other embodiments, tissue penetrating members 440 can be symmetrically or otherwise distributed around substantially the entire perimeter 430p of the balloon 430 or other expandable member 430 as is shown in the embodiments of Fig. 24a and 24b. In use, such embodiments not only anchor capsule 420 into the intestinal wall IW (as described above for bidirectional deployment) but also place tissue penetrating members 440 in a distributed pattern 440p around the circumference of the intestinal wall IW. Embodiments of the invention utilizing such a distributed delivery of drug into the intestinal wall can achieve the following: i) allow for additional amounts of a particular drug to be delivered; and ii) provide for faster absorption of the drug into the blood stream due to a more even distribution of the drug within the intestinal wall (e.g., due to placement of the tissue penetrating members within a larger volume of intestinal vascular for mass transfer and absorption into the blood).

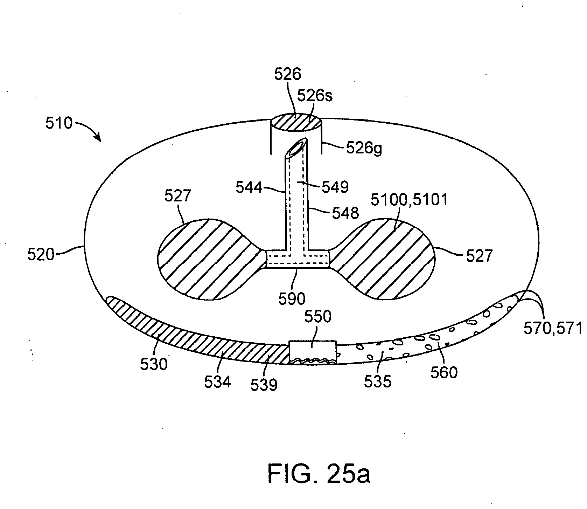

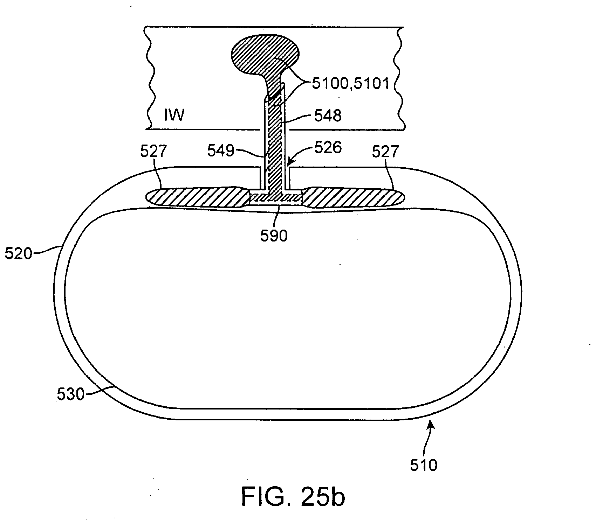

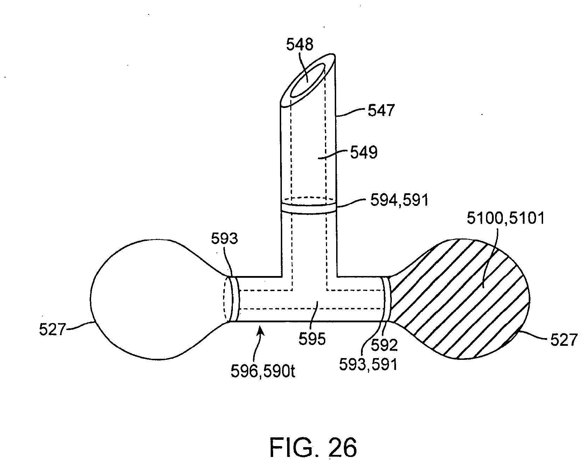

As described herein, many embodiments of device 510 include a drug carrying tissue penetrating member 540 as a means for delivering drug or other therapeutic agent 5101 into the intestinal wall. Referring now to Figs. 25a-25b and 26, as an alternative or additional means for delivering drug into the intestinal wall, in various embodiments, device 510 can also be configured to inject drug 5101 into the intestinal wall by means of hollow tissue penetrating members 548 coupled to one or more drug reservoirs 527. Hollow tissue penetrating members 548 include at least one lumen 549. Reservoirs 527 are desirably compressible by expansion of the balloon or other expandable member 530 and can thus comprise various biodegradable elastic polymers. The reservoirs 527 can contain drug or other therapeutic agent 5101 in liquid or powder form. For liquid form, the drug will be dissolved in an aqueous drug solution 5104. In these and related embodiments, reservoirs 527 are fluidically coupled to hollow tissue penetrating members 548 such that inflation of balloon 530 compresses the reservoirs 527 so as to force the drug solution 5104 through tissue penetrating member lumen 549 and into the intestinal wall as is shown in Figs. 25a and 25b. In these and related embodiments apertures 526, can include a guide tube 526g, which is horizontally aligned with the tip 544 of penetrating member 548 and configured to guide the advancement of penetrating member 548 out of capsule 520 and into the intestinal wall. Multiple reservoirs 527 are contemplated including two, three, four or more. In particular embodiments, two reservoirs 527 can be coupled to a hollow tissue penetrating member with the reservoirs placed about 180 degrees apart with respect to penetrating member shaft 544. Typically, the reservoirs 527 will be fluidically coupled to the hollow penetrating member 548 by means of a manifold 590. Suitable manifolds 590 include a t-shaped manifold 590t having connectors 592 on either of its lateral ends 593 for connection to reservoirs 527 and a central connector 594 for connection to hollow tissue penetrating member 547 and a central lumen or channel 595 going to all connectors 591 (Fig. 16). Other shapes and manifold configurations are also contemplated, for example, Y-shaped (connecting two reservoirs to tissue penetrating member 548).

In some embodiments, balloon 30 or other expandable member 30 can be expanded responsive to a sensor 67, such as a pH sensor 68 or other chemical sensor which detects the presence of the capsule in the small intestine. Sensor 67 (Fig. 1b) can then send a signal to a controllable embodiment of isolation valve 50 or to an electronic controller 29c coupled to a controllable isolation valve 50 to open and thus expand balloon 30 as is described herein. Embodiments of a pH sensor 68 can comprise an electrode-based sensor or it can be a mechanically-based sensor such as a polymer which shrinks or expands upon exposure to a selected pH or other chemical conditions in the small intestine. In related embodiments, an expandable/contractable sensor 67 can also comprise the actuating mechanism 60 itself by using the mechanical motion from the expansion or contraction of the sensor.

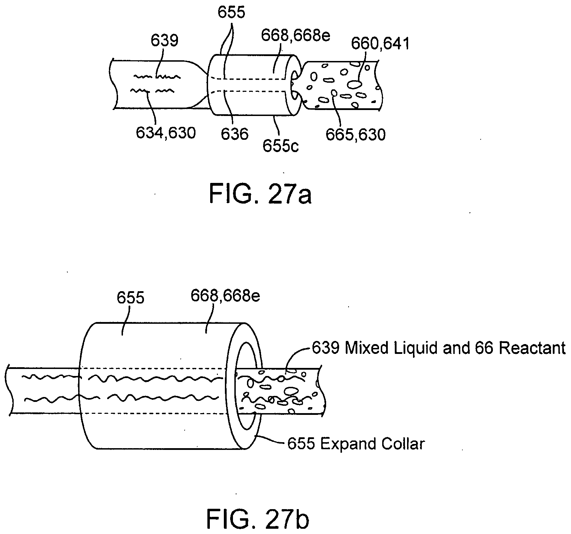

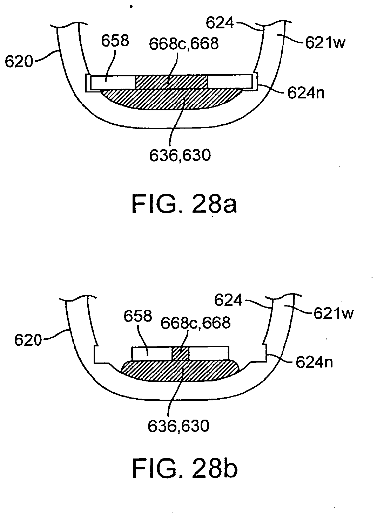

Referring now to Figs. 27a-27b and 28a-28b, in related embodiments, an expandable/contractible pH sensor 668 can also comprise the isolation valve 650 itself, by configuring the sensor to expand or contract so as to open a channel between balloon compartments 634 and 635. According to one embodiment for such an approach, a pH sensor 668 may be integrated into a collar valve 655 where sensor 668 comprises all or a portion of a collar 655c that is placed over connecting portion 636 of balloon 630 (Figs. 27a and 27b). In this embodiment, sensor 668 would be an expandable sensor 668e, configured to expand upon exposure to the pH conditions in the small intestine (e.g., a pH above 6.0, 6.5, 7.0, 7.1, 7.2, etc) so as to either have the collar come off or significantly loosen collar 655c enough to allow contents of compartments 634 and 635 to mix. According to another embodiment shown in Figs. 28a and 28b, a pH sensor 668 could be integrated into a beam valve 658 described herein, where the beam is under compressive load by being snap fit against the capsule interior surface 624. The beam applies a portion of this compressive load onto balloon connecting section 636 so as to maintain the seal between compartments 634 and 635. In this case, sensor 668 would be a contractible sensor 668c configured to open valve 650 by contracting upon exposure to higher pH in the intestine, so that the beam shortens sufficiently so that it falls out of place against capsule surface 624 or other wise no longer applies a compressive load sufficient to maintain a seal over balloon connecting section 636.

According to another embodiment for detecting when the device is in the small intestine (or other location in the GI tract), sensor 67 can comprise pressure/force sensor such as strain gauge for detecting the number of peristaltic contractions that capsule 20 is being subject to within a particular location in the intestinal tract (in such embodiments capsule 20 is desirably sized to be gripped by the small intestine during a peristaltic contraction). Different locations within the GI tract have different number of peristaltic contractions. The small intestine has between 12 to 9 contractions per minute with the frequency decreasing down the length of the intestine. Thus, according to one or more embodiments, detection of the number of peristaltic contractions can be used to not only determine if capsule 20 is in the small intestine, but the relative location within the intestine as well. In use, these and related embodiments allow for release of medication 100 at a particular location in the small intestine.

As an alternative or supplement to internally activated drug delivery (e.g., using a release element and/or sensor), in some embodiments, the user may externally send a signal to expand balloon 30 or other expandable member 30 to activate the actuating mechanism 60 to deliver medication 100 by means of RF, magnetic or other wireless signaling means known in the art. In various embodiments, including those with reference to Fig. 1b, external activation can be achieved by use of a controllable isolation valve 50 for example, a radio frequency (RF) controlled miniature solenoid valve or other electro-mechanical control valve (not shown). In other embodiments, a controllable isolation valve 50 may correspond to a miniature magnetically valve such as a magnetically controlled miniature reed switch (not shown). Such electromechanical or magnetic-based valves can be fabricated using MEMS and other micro-manufacturing methods. In these and related embodiments, the user can use a handheld communication device 13 (e.g., a hand held RF device such as a cell phone) as is shown in the embodiment of Fig, 1b, to send a receive signals 17 from device 10. In such embodiments, swallowable device may include a transmitter 28 such as an RF transceiver chip or other like communication device/circuitry. Handheld device 13 may not only includes signaling means, but also means for informing the user when device 10 is in the small intestine or other location in the GI tract. The later embodiment can be implemented through the use of logic resources 29 (e.g., a processor 29) coupled to transmitter 28 to signal to detect and singe to the user when the device is in the small intestine or other location (e.g., by signaling an input from the sensor). Logic resources 29 may include a controller 29c (either in hardware or software) to control one or more aspects of the process. The same handheld device can also be configured to alert the user when balloon 30 or actuating mechanism 60 has been expanded or activated (respectively) and the selected medication 100 delivered (e.g., using processor 29 and transmitter 28). In this way, the user is provided confirmation that medication 100 has been delivered. This allows the user to take other appropriate drugs/therapeutic agents as well as make other related decisions (e.g., for diabetics to eat a meal or not and what foods should be eaten). The handheld device can also be configured to send a signal to swallowable device 10 to over-ride isolation valve 50 or actuating mechanism 60 and so prevent delay or accelerate the delivery of medication 100. In use, such embodiments allow the user to intervene to prevent, delay or accelerate the delivery of medication, based upon other symptoms and/or patient actions (e.g., eating a meal, deciding to go to sleep, exercise etc). The user may also externally expand balloon 30 or activate actuating mechanism 60 at a selected time period after swallowing the capsule. The time period can be correlated to a typical transit time or range of transit times for food moving through the user's GI tract to a particular location in the tract such as the small intestine.

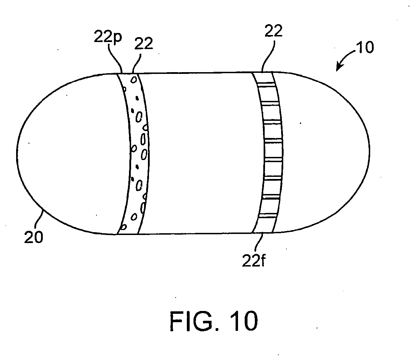

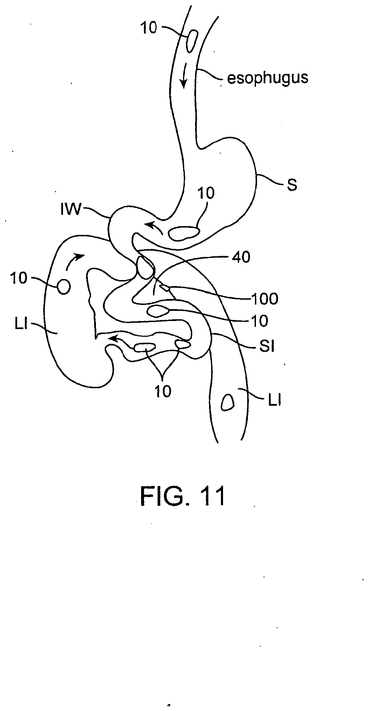

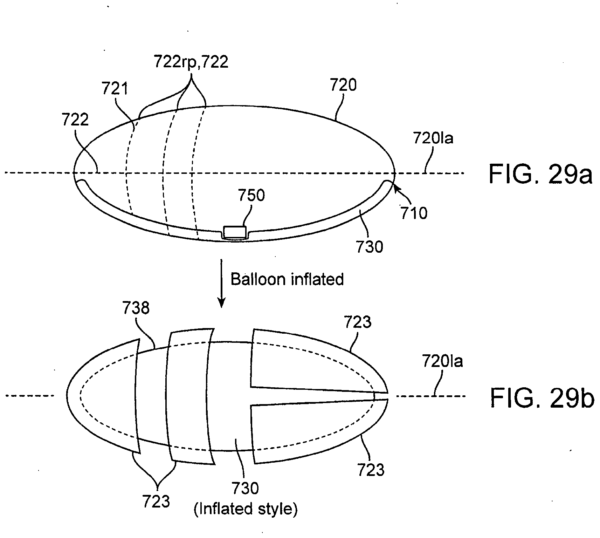

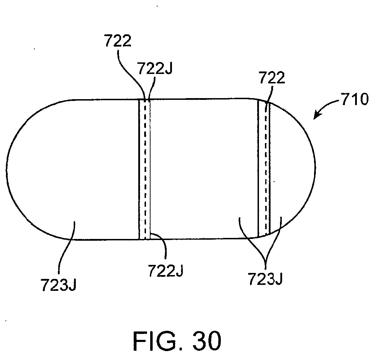

Referring now to Figs. 29a-29b and 30, in various embodiments, the capsule 720 can include seams 722 of biodegradable material which controllably degrade to produce capsule pieces 723 of a selectable size and shape to facilitate passage through the GI tract as is shown in the embodiment of Figs. 11, 29a and 29b, for example. Seams 722 can also include pores or other openings 722p for ingress of fluids into the seam to accelerate biodegradation as is shown in the embodiment of Fig. 10. Other means for accelerating biodegradation of seams 722 can include pre-stressing the seam and/or including perforations 722f in the seam (Fig. 10). In still other embodiments, seam 722 can be constructed of materials and/or have a structure which is readily degraded by absorption of ultrasound energy, e.g. high frequency ultrasound (HIFU), allowing the capsule to be degraded into smaller pieces using externally or endoscopically (or other minimally invasive method) administered ultrasound.

Referring now to Figs. 29a-29b and 30, in many embodiments seams 722 can also be configured and arranged so as to allow capsule 720 to be broken into smaller pieces by the inflation of balloon 730 or other expandable member 730. In particular embodiments, seams 722 can be oriented with respect to capsule radial perimeter 721, including having a radial pattern 722rp so as to have the capsule break into halves or other fractional pieces along its perimeter. Seams 722 may also be longitudinally-oriented with respect to capsule lateral access 7201a to have the capsule break up into lengthwise pieces.

As alternative or additional approach for breaking up capsule 720 by balloon inflation (or expansion of other expandable member 730), capsule 720 can be fabricated from two or more separate joinable pieces 723j (e.g., radial halves) that are joined at a joint 722j formed by seams 722 (which function as an adhesive joint) as shown in the embodiment of Fig. 30. Alternatively, joinable pieces 723j may be merely joined by a mechanical fit such as a snap or press fit.

Suitable materials for seams 722 can include one or more biodegradable materials described herein such as PGLA, glycolic acid etc. Seams 722 can be attached to capsule body 720 using various joining methods known in the polymer arts such as molding, hot melt junctions, etc. Additionally for embodiments of capsule 720 which are also fabricated from biodegradable materials, faster biodegradation of seam 722 can be achieved by one or more of the following: i) fabricating the seam from a faster biodegrading material, ii) pre-stressing the seam, or iii) perforating the seam. The concept of using biodegradable seams 722 to produce controlled degradation of a swallowable device in the GI tract can also be applied to other swallowable devices such as swallowable cameras (or other swallowable imaging device) to facilitate passage through the GI tract and reduce the likelihood of such a device becoming stuck in the GI tract. Accordingly, embodiments of biodegradable seam 722 can be adapted for swallowable imaging and other swallowable devices.

In still other embodiments, seam 722 can be constructed of materials and/or have a structure which is readily degraded by absorption of ultrasound energy, e.g. high frequency ultrasound (HIFU), allowing the capsule to be degraded into smaller pieces using externally or endoscopically (or other minimally invasive method) administered ultrasound.

Further are disclosed, not claimed, methods for the delivery of drugs and other therapeutic agents (in the form of medication 100) into the walls of the GI tract using one or more embodiments of swallowable drug delivery device 10. An example of such a method will now be described. The described example of drug delivery occurs in the small intestine SI. However, it should be appreciated that this is exemplary and that methods can be used for delivering drug in a number of locations in the GI tract including the stomach and the large intestine. For ease of discussion, the swallowable drug delivery device 10 will sometimes be referred to herein as a capsule. As described above, in various embodiments device 10 may be packaged as a kit 11 within sealed packaging 12 that includes device 10 and a set of instructions for use 15. If the patient is using a handheld device 13, the patient may instructed to enter data into device 13 either manually or via a bar code 18 (or other identifying indicia 18) located on the instructions 15 or packaging 12. If a bar code is used, the patient would scan the bar code using a bar code reader 19 on device 13. After opening packaging 12, reading the instructions 15 and entering any required data, the patient swallows an embodiment of the swallowable drug delivery device 10. Depending upon the drug, the patient may take the device 10 in conjunction with a meal (before, during or after) or a physiological measurement such as a blood glucose measurement. Capsule 20 is sized to pass through the GI tract and travels through the patient's stomach S and into the small intestine SI through peristaltic action as is shown in the embodiment of Fig. 11. Once the capsule 10 is in the small intestine, the release element 70 is degraded by the basic pH in the small intestine (or other chemical or physical condition unique to the small intestine) so as expand balloon 30 or other expandable member 30, actuate the actuating mechanism 60 and deliver medication 100 into the wall of the small intestine SI. In case a hollow needle or other hollow tissue penetrating member 40 is used, medication delivery is effectuated by using balloon 30 the actuating mechanism 60 to advance the needle 40 a selected distance into the mucosa of the intestinal wall IS, and then the medication is injected through the needle lumen by advancement of the delivery member 50. The delivery member 50 is withdrawn and the needle 40 is then withdrawn back within the body of the capsule (e.g. by recoil) detaching from the intestinal wall. For embodiments of device 10 having multiple needles, a second or third needle 42, 43 can also be used to deliver additional doses of the same drug or separate drugs 101. Needle advancement can be done substantially simultaneously or in sequence. When using multiple needles, needle advancement can be done substantially simultaneously so as to anchor device 10 in the small intestine during drug delivery.

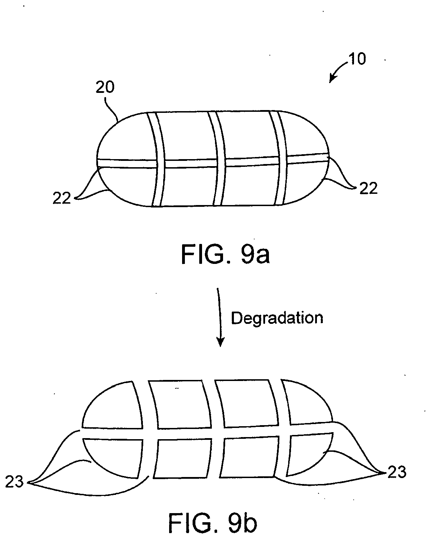

After medication delivery, device 10 then passes through the intestinal tract including the large intestine LI and is ultimately excreted. When using a tearable capsule, the capsule may immediately be broken into smaller pieces by inflation of balloon 30. For embodiments of the capsule 20 having biodegradable seams 22 or other biodegradable portions, the capsule is degraded in the intestinal tract into smaller pieces, to facilitate passage through and excretion from the intestinal tract as is shown in Figs. 9a and 9b. When using biodegradable tissue penetrating needles/members 40, should the needle get stuck in the intestinal wall, the needle biodegrades releasing the capsule 20 from the wall.

For embodiments of device 10 including a sensor 67, can be effectuated by the senor sending a signal to a controllable embodiment of isolation valve 50 or actuating mechanism 60 and/or a processor 29/controller 29c coupled to the isolation valve 50 or actuating mechanism. For embodiments of device 10 including external actuation capability, the user may externally expand balloon 30 or activate actuating mechanism 60 at a selected time period after swallowing the capsule. The time period can be correlated to a typical transit time or range of transit times for food moving through the user's GI tract to a particular location in the tract such as the small intestine.

One or more of the above methods can be used for the delivery of preparations 100 containing therapeutically effective amounts of a variety of drugs and other therapeutic agents 101 to treat a variety of diseases and conditions. These include a number of large molecule peptides and proteins which would otherwise require injection and/or IV infusion due to chemical breakdown or other degradation of the compound by the digestive fluids in the stomach and/or the lumen of the small intestine. Such compounds which can be delivered can include without limitation, parathyroid hormones, growth hormones (e.g., IFG and other growth factors), insulin compounds, antibodies and other gamma globulin proteins (e.g., gamma globulin) interferons and other cytokines, glucagon like peptides e.g., (GLP-1, exenatide) and other incretins, chemotherapeutic agents (doxorubicin) and other like compounds. These and other compounds can be delivered into the wall of the small intestine and subsequently absorbed into the blood stream with minimal or no loss of activity of the compound, e.g., in the case of an antibody, minimal or no loss in affinity and/or specificity to a target antigen; in the case of an interferon or other cytokine, minimal or no loss in an immune stimulating effect, in the case of insulin or GLP-1, minimal or no loss in glucose regulating ability; in the case of growth hormone, minimal or no loss in growth stimulating effect; in the case of a chemotherapeutic agent for the treatment of cancer, minimal or no loss in cancer treatment effect (e.g., a tumor necrosis, and/or reduced cell division); and in the case of any polypeptide, minimal or no loss in affinity and/or specificity to a target binding site. Suitable drugs and other therapeutic agents which can be delivered by embodiments of the invention include any number of orally delivered agents, antibiotics (vancomycin, penicillin, erythromycin, etc.), antivirals (protease inhibitors, anti-seizure compounds (fluosemide, dilatin), non-steroidal anti-inflamatory drugs (NSAIDS) such as ibuprofen), various chemotherapeutic agents (e.g., interferon), antibiotics, antivirals, insulin and related compounds, glucagon like peptides (e.g., GLP-1, exenatide), parathyroid hormones, growth hormones (e.g., IFG and other growth factors), anti-seizure agents (e.g., furosimide), anti-migraine medication (sumatriptan), immune suppression agents (e.g., cyclosporine) and anti- parasitic agents such as various anti- malarial agents. The dosage of the particular drug can be titrated for the patient's weight, age or other parameter. It is also possible to allow dosages of drug other therapeutic agent 101 to be advantageously adjusted for other factors as well. For example, for drugs that would otherwise be partially degraded or poorly absorbed in the GI tract, the amount or dose of drug 101 to achieve a desired or therapeutic effect (e.g., insulin for blood glucose regulation, furosimide for anti-seizure) can be less than the amount required should the drug have been delivered by conventional oral delivery (e.g., a swallowable pill that is digested in the stomach and absorbed through the wall of the small intestine). This is due to the fact that there is little or no degradation of the drug by acid and other digestive fluids in the stomach and the fact that all, as opposed to only a portion of the drug is delivered into the wall of the small intestine (or other lumen in the intestinal tract, e.g., large intestine, stomach, etc.). Depending upon the drug 101, the dose 102 delivered in preparation 100 can be in the range from 100 to 5% of a dose delivered by conventional oral delivery means (e.g., a formulated pill) to achieve a desired therapeutic effect (e.g., blood glucose regulation, seizure regulation, etc.) with even lower amounts contemplated. The particular dose reduction can be titrated based upon the particular drug, the condition to be treated, and the patient's weight, age and condition. For some drugs (with known levels of degradation in the intestinal tract) a standard dose reduction can be employed (e.g., 10 to 20%). Larger amounts of dose reduction can be used for drugs which are more prone to degradation in the GI tract and poor absorption. In this way, the potential toxicity (particularly to non target tissue sites) and other other deleterious side effects (e.g., gastric cramping, diarrhea, irritable bowel, hemorrhage, etc.) of a particular drug or drugs delivered by device 10 can be reduced because the ingested dose is lowered and all or nearly all of the drug is delivered into the wall of the small intestine. This in turn, improves patient compliance because the patient has a reduction both in the severity and incidence of deleterious effects. Additional benefits of dose reduction of drug 101 that include a reduced likelihood for the patient to develop a tolerance to the drug (requiring higher doses) and, in the case of antibiotics or antivirals, for the patient to develop resistant strains of bacteria or viruses (e.g., resistance to the use of vancomycin by bacteria or to a protease inhibitor by the Aids virus). For the case of a chemotherapeutic agent for the treatment of cancer, the deleterious effect can comprise the development of resistance to the chemotherapeutic agent by cancer cells as well as toxicity to non-target tissue. For the case of an anti-seizure medication such as dilatin, the deleterious effects can include various neuromuscular conditions such as tremor, nystagmus, slurred speech, dizziness, memory and concentration problems as well conditions such as rash and bone loss. For anti-seizure and/or diuretics such as furesomide such deleterious effects can include various neuromuscular, vascular, gastro intestinal effects such as dizziness, low blood pressure, dehydration, nausea, loss of electrolytes, tinnitus and rash. Also, other levels of dose reduction can be achieved for patients who have undergone gastric bypass surgery and other procedures in which sections of the small intestine have been removed or its working (e.g., digestive) length otherwise effectively shortened. Levels of dose reduction can be achieved in the range of 25 to 50% or even greater and the patient need only take one dose of the drug versus multiple doses because of poor absorption issues. The dose of a particular orally delivered drug 101 can be increased because the various deleterious effects in the GI system (e.g., cramping, bleeding, etc.) are avoided since the drug or other therapeutic agent is injected directly into the wall of the small intestine. This increased dosage in turn allows for one or more of the following: fewer doses, faster treatment, faster obtainment of a therapeutic effective level of the drug in the blood stream , better control of blood concentrations and other pharmacokinetic parameters. The dosage of a particular drug can increased in the range of 5 to 100% or higher. The amount of the increase can again be titrated based on the patient's, weight, age, condition and individual tolerance to the drug (which can be determined e.g., by using various biomarkers of tolerance and/or toxicity).

In addition to delivery of a single drug, embodiments of swallowable drug delivery device 10 can be used to deliver a plurality of drugs for the treatment of multiple conditions or for the treatment of a particular condition (e.g., protease inhibitors for treatment HIV AIDS). This allow a patient to forgo the necessity of having to take multiple medications for a particular condition or conditions. Also, they provide a means for facilitating that a regimen of two or more drugs is delivered and absorbed into the small intestine and thus, the blood stream, at about the same time. Due to difference in chemical makeup, molecular weight, etc, drugs can be absorbed through the intestinal wall at different rates, resulting in different pharmacokinetic distribution curves. It is possible to address this issue by injecting the desired drug mixtures at substantially the same time. This in turn, improves the pharmacokinetics and thus the efficacy of the selected mixture of drugs. Additionally, eliminating the need to take multiple drugs is particularly beneficial to patients who have one or more long term chronic conditions including those who have impaired cognitive or physical abilities.

In various applications, the above methods can be used to deliver preparations 100 including drugs and therapeutic agents 101 to provide treatment for a number of medical conditions and diseases. The medical conditions and diseases which can be treated can include without limitation: cancer, hormonal conditions (e.g., hypo/hyper thyroid, growth hormone conditions), osteoporosis, high blood pressure, elevated cholesterol and triglyceride, diabetes and other glucose regulation disorders, infection (local or septicemia), epilepsy and other seizure disorders, osteoporosis, coronary arrhythmia's (both atrial and ventricular), coronary ischemia anemia or other like condition. Still other conditions and diseases are also contemplated.

The treatment of the particular disease or condition can be performed without the need for injecting the drug or other therapeutic agent (or other non-oral form of delivery such as suppositories) but instead, relying solely on the therapeutic agent(s) that is delivered into the wall of the small intestine or other portion of the GI tract. For example, diabetes or another glucose regulation disorder can be treated (e.g., by controlling blood glucose levels) solely through the use of insulin that is delivered into the wall of the small intestine without the need for the patient to ever inject insulin. Similarly, the patient need not take conventional oral forms of a drug or other therapeutic agent, but again rely solely on delivery into the wall of the small intestine using embodiments of the swallowable capsule. In other instances, the therapeutic agent(s) delivered into the wall of the small intestine can be delivered in conjunction with an injected dose of the agent(s). For example, the patient may take a daily dose of insulin or compound for blood glucose regulation using the embodiments of the swallowable capsule, but only need take an injected dose every several days or when the patient's condition requires it (e.g., hyperglycemia). The same is true for therapeutic agents that are traditionally delivered in oral form (e.g., the patient can take the swallowable capsule and take the conventional oral form of the agent as needed). The dosages delivered
(e.g., the swallowed and injected dose) can be titrated as needed (e.g., using standard dose response curve and other pharmacokinetic methods can be used to determine the appropriate dosages). Also, when using therapeutic agents that can be delivered by conventional oral means, the dose delivered using embodiments of the swallowable capsule can be titrated below the dosage normally given for oral delivery of the agent since there is little or no degradation of the agent within the stomach or other portion of the intestinal tract (herein again standard dose response curve and other pharmacokinetic methods can be applied).

Various groups of embodiments of preparation 100 containing one or more drugs or other therapeutic agents 101 for the treatment of various diseases and conditions will now be described with references to dosages. It should be appreciated that these embodiments, including the particular therapeutic agents and the respective dosages are exemplary and the preparation 100 can comprise a number of other therapeutic agents described herein (as well as those known in the art) that are configured for delivery into a luminal wall in the intestinal tract (e.g., the small intestinal wall) using various embodiments of device 10. The dosages can be larger or smaller than those described and can be adjusted using one or more methods described herein or known in the art. In one group of embodiments, therapeutic agent preparation 100 can comprise a therapeutically effective dose of insulin for the treatment of diabetes and other glucose regulation disorders. The insulin can be human or synthetically derived as is known in the art. In one embodiment, preparation 100 can contain a therapeutically effective amount of insulin in the range of about 1-10 units (one unit being the biological equivalent of about 45.5 µg of pure crystalline insulin), with particular ranges of 2-4, 3-9, 4-9, 5-8 or 6-7. The amount of insulin in the preparation can be titrated based upon one or more of the following factors (herein, then 'glucose control titration factors"): i) the patient's condition (e.g., type 1 vs. type II diabetes; ii) the patients previous overall level of glycemic control; iii) the patient's weight; iv) the patient's age; v) the frequency of dosage (e.g., once vs. multiple times a day); vi) time of day (e.g., morning vs. evening); vii) particular meal (breakfast vs. dinner); viii) content/glycemic index of a particular meal (e.g., meals having a high fat/lipid and sugar content (which tend to cause a rapid rise in blood sugar and thus have a higher glycemic index) vs. low fat and sugar content that do not (and thus have a lower glycemic index)); and ix) content of the patient's overall diet (e.g., amount of sugars and other carbohydrates, lipids and protein consumed daily).

In another group of embodiments, therapeutic agent preparation 100 can comprise a therapeutically effective dose of one or more incretins for the treatment of diabetes and other glucose regulation disorders. Such incretins can include Glucacon like peptides 1 (GLP-1) and their analogues, and Gastric inhibitory peptide (GIP). Suitable GLP-1 analogues include exenatide, liraglutide, albiglutide and taspoglutide as well as their analogues, derivatives and other functional equivalents. In one embodiment preparation 100 can contain a therapeutically effective amount of exenatide in the range of about 1-10 µg, with particular ranges of 2-4, 4-6, 4-8 and 8-10 µg respectively. In another embodiment, preparation 100 can contain a therapeutically effective amount of liraglutide in the range of about 1-2 mg (milligrams), with particular ranges of 1.0 to 1.4, 1.2 to 1.6 and 1.2 to 1.8 mg respectively. One or more of the glucose control titration factors can be applied to titrate the dose ranges for exenatide, liraglutide or other GLP-1 analogue or incretin.

In yet another group of embodiments, therapeutic agent preparation 100 can comprise a combination of therapeutic agents for the treatment of diabetes and other glucose regulation disorders. Embodiments of such a combination can include therapeutically effective doses of incretin and biguanide compounds. The incretin can comprise one or more GLP-1 analogues described herein, such as exenatide and the biguanide can comprise metformin (e.g., that available under the Trademark of GLUCOPHAGE^{®} manufactured by Merck Santé S.A.S.) and its analogues, derivatives and other functional equivalents. In one embodiment, preparation 100 can comprise a combination of a therapeutically effective amount of exenatide in the range of about 1-10 µg and a therapeutically effective amount of metformin in a range of about 1 to 3 grams. Smaller and larger ranges are also contemplated with one or more of the glucose control titration factors used to titrate the respective dose of exenatide (or other incretin) and metformin or other biguanide. Additionally, the dosages of the exenatide or other incretin and metformin or other biguanide can be matched to improve the level of glucose control for the patient (e.g., maintenance of blood glucose within normal physiological levels and/or a reduction in the incidence and severity of instances of hyperglycemia and/or hypoglycemia) for extended periods of time ranging from hours (e.g., 12) to a day to multiple days, with still longer periods contemplated. Matching of dosages can also be achieved by use of the glucose control regulation factors as well as monitoring of the patient's blood glucose levels for extended periods using glycosylated hemoglobin (known as hemoglobin A1c, HbA1c, A1C, or Hb1c) and other analytes and measurements correlative to long term average blood glucose levels.

In still yet another group of embodiments, therapeutic agent preparation 100 can comprise a therapeutically effective dose of growth hormone for the treatment of one or more growth disorders, as well as wound healing. In one embodiment, preparation 100 can contain a therapeutically effective amount of growth hormone in the range of about 0.1-4 mg, with particular ranges of 0.1-1, 1-4, 1-2 and 2-4, with still larger ranges contemplated. The particular dose can be titrated based on one or more of the following: i) the particular condition to be treated and its severity (e.g., stunted growth, vs. wound healing); ii) the patient's weight; iii) the patient's age; and iv) the frequency of dosage (e.g., daily vs. twice daily).

In still yet another group of embodiments, therapeutic agent preparation 100 can comprise a therapeutically effective dose of parathyroid hormone for the treatment osteoporosis or a thyroid disorder. In one embodiment, preparation 100 can contain a therapeutically effective amount of parathyroid hormone in the range of about 1-40 µg, with particular ranges of 10-20, 20-30, 30-40 and 10-40 µg, with still larger ranges contemplated. The particular dose can be titrated based on one or more of the following: i) the particular condition to be treated and its severity (e.g., the degree of osteoporosis as determined by bone density measurements); ii) the patient's weight; iii) the patient's age; and iv) the frequency of dosage (e.g., daily vs. twice daily).

The foregoing description of various embodiments of the invention has been presented for purposes of illustration and description. It is not intended to limit the invention to the precise forms disclosed. Many modifications, variations and refinements will be apparent to practitioners skilled in the art. For example, embodiments of the device can be sized and otherwise adapted for various pediatric and neonatal applications as well as various veterinary applications.

Elements, characteristics, or acts from one embodiment can be readily recombined or substituted with one or more elements, characteristics or acts from other embodiments to form numerous additional embodiments within the scope of the invention. Moreover, elements that are shown or described as being combined with other elements, can, in various embodiments, exist as standalone elements. Hence, the scope of the present invention is not limited to the specifics of the described embodiments, but is instead limited solely by the appended claims.

## Claims

1. An ingestible device (10) suitable for swallowing into a lumen of an intestinal tract of a patient, the lumen having a lumen wall, the device comprising:
a capsule (20) sized to pass through the intestinal tract;
a therapeutic agent preparation (100) disposed within the capsule, the preparation comprising at least one therapeutic agent (101), wherein the therapeutic agent preparation would chemically degrade or impose a deleterious effect on the patient if released within the lumen of the intestinal tract, wherein the therapeutic agent preparation further comprises a tissue penetrating member (40) including a hollow needle that is advanceable into the lumen wall, wherein the hollow needle is formed from a biodegradable material so as to degrade within the lumen wall; and
an actuator (70a) coupled to the therapeutic agent preparation and having a first configuration and a second configuration, the preparation being retained within the capsule when the actuator is in the first configuration, wherein the preparation is advanced from the capsule and into the lumen wall by movement of the actuator from the first configuration to the second configuration such that the deleterious effect or chemical degradation of the therapeutic agent in the lumen is inhibited, wherein a therapeutic dose of the therapeutic agent is included in the capsule, and wherein the actuator is configured to advance substantially all of the therapeutic dose of the therapeutic agent into the lumen wall by way of the hollow needle so as to minimize the deleterious effect.

2. The device of claim 1, wherein:
i) the preparation being retained entirely within the capsule when the actuator is in the first configuration; and/or
ii) the actuator includes a release element (70) comprising a material configured to degrade upon exposure to a selected pH in the intestinal tract such that upon degradation, the preparation is advanced into the lumen wall.

3. The device of claim 2 wherein the selected pH is a pH in the small intestine.

4. The device of claim 2, wherein the actuator comprises a spring (80), the release element being coupled to the spring to retain the spring in a compressed state (85) and release the spring upon degradation of the release element.

5. The device of claim 1, wherein the therapeutic agent preparation includes at least a first therapeutic agent and a second therapeutic agent.

6. The device of claim 1, wherein the therapeutic agent preparation comprises one of:-
a) a therapeutically effective dose of insulin for the treatment of diabetes or a glucose regulation disorder, or
b) a therapeutically effective dose of an incretin for the treatment of diabetes or a glucose regulation disorder, optionally wherein the incretin comprises a glucagon like peptide-1 (GLP-1), a GLP-1 analogue, exenatide, liraglutide, albiglutide, taspoglutide or a gastric inhibitory polypeptide (GIP), or
c) a combination of therapeutic agents for the treatment of diabetes or a glucose regulation disorder, optionally, wherein the combination comprises a therapeutically effective dose of an incretin and a therapeutically effective dose of a biguanide, optionally:
i) wherein the incretin comprises exenatide and the biguanide comprises metformin, or
ii) wherein the dosages of the incretin and the biguanide are matched to produce an improved level of blood glucose control for an extended period, or
d) a therapeutically effective dose of growth hormone, or
e) a therapeutically effective dose of parathyroid hormone for the treatment of osteoporosis or a thyroid disorder, or
f) a therapeutically effective dose of a chemotherapeutic agent for the treatment of cancer, or
g) a therapeutically effective dose of antibiotic, or
h) a therapeutically effective dose of an antiviral compound, optionally wherein the antiviral compound comprises a protease inhibitor, or
i) a therapeutically effective dose of an anti-seizure compound, optionally wherein the anti-seizure compound comprises furosemide.

7. The therapeutic agent preparation of claim 6, wherein the therapeutically effective doses of insulin, exenatide, liraglutide, growth hormone, and parathyroid hormone are in the range of:
a) for insulin, about 1-10 units, optionally in the range of 2-4, 3-9, 4-9, 5-8 or 6-7 units, wherein one unit is the biological equivalent of about 45.5 µg of pure crystalline insulin;
b) for exenatide, about 1-10 µg, optionally in the range of 2-4, 4-6, 4-8 and 8-10 µg;
c) for liraglutide, about 1-2 mg, optionally in the range of 1.0 to 1.4, 1.2 to 1.6 and 1.2 to 1.8 mg;
e) for growth hormone, about 0.1-4 mg, optionally in the range of 0.1-1, 1-4, 1-2 and 2-4 mg; and
f) for parathyroid hormone, about 1-40 µg, optionally in the range of 10-20, 20-30, 30-40 and 10-40 µg.

8. The therapeutically effective dose of insulin in claim 6 or 7, wherein the insulin is human or synthetically derived.

9. The combination of therapeutic agents of claim 6, part i), wherein the therapeutically effective dose of exenatide is in the range of about 1-10 µg and the therapeutically effective dose of metformin is in the range of about 1-3 g.

10. The device of claim 1, wherein the capsule is formed from a biodegradable material so as to degrade in whole or in part in the intestinal tract.

11. The device of claim 1 or 10, wherein the capsule includes an enteric or other coating for protecting the capsule from stomach acids while allowing for biodegradation in the small intestine.

12. The device of claim 1, wherein the actuator further comprises an expandable member.

13. The device of claim 12, wherein the expandable member comprises a balloon, wherein the balloon includes a first compartment and a second compartment which are separated by a separation valve, wherein a liquid is disposed in the first compartment and at least one reactant is disposed in the second compartment.

14. The device of claim 13, wherein the separation valve is configured to degrade in response to a pH of the small intestine so that upon degradation, the valve opens.

15. The device of claim 13 or 14, wherein when the valve opens, the at least one reactant mixes in the liquid to produce a gas to expand the balloon and advance the tissue penetrating member into the lumen wall.

## Patentansprüche

1. Einnehmbare Vorrichtung (10), die zum Schlucken in ein Lumen eines Darmtrakts eines Patienten geeignet ist, wobei das Lumen eine Lumenwand aufweist, wobei die Vorrichtung Folgendes umfasst:
eine Kapsel (20), die zum Durchgang durch den Darmtrakt dimensioniert ist;
ein Therapeutikum-Präparat (100), das innerhalb der Kapsel angeordnet ist, wobei das Präparat mindestens ein Therapeutikum (101) umfasst, wobei das Therapeutikum-Präparat chemisch abgebaut werden würde oder einen nachteiligen Effekt auf den Patienten ausüben würde, wenn es innerhalb des Lumens des Darmtrakts freigesetzt wird, wobei das Therapeutikum-Präparat weiter ein gewebedurchdringendes Element (40) umfasst, das eine Hohlnadel einschließt, die in die Lumenwand vorgeschoben werden kann, wobei die Hohlnadel aus einem biologisch abbaubaren Material ausgebildet ist, sodass sie innerhalb der Lumenwand abgebaut wird; und
einen Auslöser (70a), der mit dem Therapeutikum-Präparat verbunden ist und eine erste Konfiguration und eine zweite Konfiguration aufweist, wobei das Präparat innerhalb der Kapsel zurückgehalten wird, wenn der Auslöser in der ersten Konfiguration vorliegt, wobei das Präparat aus der Kapsel und in die Lumenwand durch Bewegung des Auslösers aus der ersten Konfiguration in die zweite Konfiguration vorgeschoben wird, sodass der nachteilige Effekt oder chemische Abbau des Therapeutikums in dem Lumen verhindert wird, wobei eine therapeutische Dosis des Therapeutikums in der Kapsel eingeschlossen ist und wobei der Auslöser konfiguriert ist, um im Wesentlichen die gesamte therapeutische Dosis des Therapeutikums in die Lumenwand mithilfe der Hohlnadel vorzuschieben, sodass der nachteilige Effekt minimiert wird.

2. Vorrichtung nach Anspruch 1, wobei:
i) das Präparat vollständig innerhalb der Kapsel zurückgehalten wird, wenn der Auslöser in der ersten Konfiguration vorliegt; und/oder
ii) der Auslöser ein Freisetzungselement (70) einschließt, das ein Material umfasst, das bei Aussetzung gegenüber einem ausgewählten pH in dem Darmtrakt zum Abbau konfiguriert ist, sodass bei Abbau das Präparat in die Lumenwand vorgeschoben wird.

3. Vorrichtung nach Anspruch 2, wobei der ausgewählte pH ein pH in dem Dünndarm ist.

4. Vorrichtung nach Anspruch 2, wobei der Auslöser eine Feder (80) umfasst, wobei das Freisetzungselement mit der Feder verbunden ist, um die Feder in einem komprimierten Zustand (85) zu halten und die Feder bei Abbau des Freisetzungselements freizusetzen.

5. Vorrichtung nach Anspruch 1, wobei das Therapeutikum-Präparat mindestens ein erstes Therapeutikum und ein zweites Therapeutikum einschließt.

6. Vorrichtung nach Anspruch 1, wobei das Therapeutikum-Präparat eine von Folgenden umfasst:
a) eine therapeutisch wirksame Dosis von Insulin für die Behandlung von Diabetes oder einer Störung der Glucoseregulation oder
b) eine therapeutisch wirksame Dosis eines Inkretins für die Behandlung von Diabetes oder einer Störung der Glucoseregulation, optional wobei das Inkretin ein Glucagon-like Peptide-1 (GLP-1), ein GLP-1-Analogon, Exenatid, Liraglutid, Albiglutid, Taspoglutid oder ein Gastric Inhibitory Polypeptide (GIP) umfasst, oder
c) eine Kombination aus Therapeutika für die Behandlung von Diabetes oder einer Störung der Glucoseregulation, optional wobei die Kombination eine therapeutisch wirksame Dosis eines Inkretins und eine therapeutisch wirksame Dosis eines Biguanids umfasst, optional:
i) wobei das Inkretin Exenatid umfasst und das Biguanid Metformin umfasst oder
ii) wobei die Dosierungen des Inkretins und des Biguanids abgestimmt sind, um ein verbessertes Niveau der Blutzuckerkontrolle für einen längeren Zeitraum zu erzeugen, oder
d) eine therapeutisch wirksame Dosis an Wachstumshormon oder
e) eine therapeutisch wirksame Dosis an Parathormon für die Behandlung von Osteoporose oder einer Schilddrüsenstörung oder
f) eine therapeutisch wirksame Dosis eines Chemotherapeutikums für die Behandlung von Krebs oder
g) eine therapeutisch wirksame Dosis von Antibiotikum oder
h) eine therapeutisch wirksame Dosis einer antiviralen Verbindung, optional wobei die antivirale Verbindung einen Proteaseinhibitor umfasst, oder
i) eine therapeutisch wirksame Dosis einer antikonvulsiven Verbindung, optional wobei die antikonvulsive Verbindung Furosemid umfasst.

7. Therapeutikum-Präparat nach Anspruch 6, wobei die therapeutisch wirksamen Dosen von Insulin, Exenatid, Liraglutid, Wachstumshormon und Parathormon in dem folgenden Bereich liegen:
a) für Insulin etwa 1-10 Einheiten, optional in dem Bereich von 2-4, 3-9, 4-9, 5-8 oder 6-7 Einheiten, wobei eine Einheit das biologische Äquivalent von etwa 45,5 µg von reinem kristallinem Insulin ist;
b) für Exenatid etwa 1-10 µg, optional in dem Bereich von 2-4, 4-6, 4-8 und 8-10 µg;
c) für Liraglutid etwa 1-2 mg, optional in dem Bereich von 1,0 bis 1,4, 1,2 bis 1,6 und 1,2 bis 1,8 mg;
d) für Wachstumshormon etwa 0,1-4 mg, optional in dem Bereich von 0,1-1, 1-4, 1-2 und 2-4 mg; und
e) für Parathormon etwa 1-40 µg, optional in dem Bereich von 10-20, 20-30, 30-40 und 10-40 µg.

8. Therapeutisch wirksame Dosis von Insulin nach Anspruch 6 oder 7, wobei das Insulin menschlich oder synthetisch hergestellt ist.

9. Kombination aus Therapeutika nach Anspruch 6, Abschnitt i), wobei die therapeutisch wirksame Dosis von Exenatid in dem Bereich von etwa 1-10 µg liegt und die therapeutisch wirksame Dosis von Metformin in dem Bereich von etwa 1-3 g liegt.

10. Vorrichtung nach Anspruch 1, wobei die Kapsel aus einem biologisch abbaubaren Material ausgebildet ist, sodass sie ganz oder teilweise in dem Darmtrakt abgebaut wird.

11. Vorrichtung nach Anspruch 1 oder 10, wobei die Kapsel eine enterische oder andere Beschichtung zum Schutz der Kapsel vor Magensäuren einschließt, während der biologische Abbau in dem Dünndarm ermöglicht ist.

12. Vorrichtung nach Anspruch 1, wobei der Auslöser weiter ein expandierbares Element umfasst.

13. Vorrichtung nach Anspruch 12, wobei das expandierbare Element einen Ballon umfasst, wobei der Ballon eine erste Kammer und eine zweite Kammer einschließt, die durch ein Trennventil getrennt sind, wobei eine Flüssigkeit in der ersten Kammer angeordnet ist und mindestens ein Reaktionsmittel in der zweiten Kammer angeordnet ist.

14. Vorrichtung nach Anspruch 13, wobei das Trennventil zum Abbau als Reaktion auf einen pH des Dünndarms konfiguriert ist, um das Ventil bei Abbau zu öffnen.

15. Vorrichtung nach Anspruch 13 oder 14, wobei bei Öffnung des Ventils das mindestens eine Reaktionsmittel in die Flüssigkeit gemischt wird, um ein Gas zu erzeugen, das den Ballon expandiert, und das gewebedurchdringende Element in die Lumenwand vorzuschieben.

## Revendications

1. Dispositif ingérable (10) approprié pour être avalé dans une lumière d'un tractus intestinal d'un patient, la lumière comportant une paroi de lumière, le dispositif comprenant :
une capsule (20) dimensionnée pour passer à travers le tractus intestinal ;
une préparation d'agent thérapeutique (100) disposée à l'intérieur de la capsule, la préparation comprenant au moins un agent thérapeutique (101), dans lequel la préparation d'agent thérapeutique se dégraderait chimiquement ou imposerait un effet délétère au patient si elle était libérée à l'intérieur de la lumière du tractus intestinal, la préparation d'agent thérapeutique comprenant en outre un élément de pénétration de tissu (40) incluant une aiguille creuse qui peut être avancée dans la paroi de la lumière, l'aiguille creuse étant formée à partir d'un matériau biodégradable de sorte à se dégrader à l'intérieur de la paroi de la lumière ; et
un actionneur (70a) accouplé à la préparation d'agent thérapeutique et ayant une première configuration et une seconde configuration, la préparation étant retenue à l'intérieur de la capsule quand l'actionneur est dans la première configuration, la préparation étant avancée depuis la capsule et dans la paroi de la lumière par un mouvement de l'actionneur de la première configuration à la seconde configuration de telle sorte que l'effet délétère ou la dégradation chimique de l'agent thérapeutique dans la lumière soit inhibé, une dose thérapeutique de l'agent thérapeutique étant incluse dans la capsule, et l'actionneur étant conçu pour avancer sensiblement la totalité de la dose thérapeutique de l'agent thérapeutique dans la paroi de la lumière au moyen de l'aiguille creuse de sorte à réduire au minimum l'effet délétère.

2. Dispositif selon la revendication 1, dans lequel :
i) la préparation est entièrement retenue à l'intérieur de la capsule quand l'actionneur est dans la première configuration ; et/ou
ii) l'actionneur inclut un élément de libération (70) comprenant un matériau conçu pour se dégrader lorsqu'il est exposé à un pH sélectionné dans le tractus intestinal de telle sorte que lors de la dégradation, la préparation soit avancée dans la paroi de la lumière.

3. Dispositif selon la revendication 2, dans lequel le pH sélectionné est un pH de l'intestin grêle.

4. Dispositif selon la revendication 2, dans lequel l'actionneur comprend un ressort (80), l'élément de libération étant accouplé au ressort pour retenir le ressort dans un état comprimé (85) et libérer le ressort lors de la dégradation de l'élément de libération.

5. Dispositif selon la revendication 1, dans lequel la préparation d'agent thérapeutique comprend au moins un premier agent thérapeutique et un deuxième agent thérapeutique.

6. Dispositif selon la revendication 1, dans lequel la préparation d'agent thérapeutique comprend l'une :
a) d'une dose thérapeutiquement efficace d'insuline destinée au traitement du diabète ou d'un trouble de la régulation du glucose, ou
b) d'une dose thérapeutiquement efficace d'une incrétine destinée au traitement du diabète ou d'un trouble de la régulation du glucose, éventuellement l'incrétine comprenant un peptide de type glucagon-1 (GLP-1), un analogue de GLP-1, de l'exénatide, du liraglutide, de l'albiglutide, du taspoglutide ou un polypeptide inhibiteur gastrique (GIP), ou
c) d'une combinaison d'agents thérapeutiques destinés au traitement du diabète ou d'un trouble de la régulation du glucose, éventuellement, la combinaison comprenant une dose thérapeutiquement efficace d'une incrétine et une dose thérapeutiquement efficace d'un biguanide, éventuellement :
i) l'incrétine comprenant l'exénatide et le biguanide comprenant la métformine, ou
ii) les dosages de l'incrétine et du biguanide sont associés pour produire un niveau amélioré de la régulation du glucose sanguin pendant une période étendue, ou
d) d'une dose thérapeutiquement efficace d'hormone de croissance, ou
e) d'une dose thérapeutiquement efficace de parathormone destinée au traitement de l'ostéoporose ou d'un trouble de la thyroïde, ou
f) d'une dose thérapeutiquement efficace d'un agent chimiothérapeutique destiné au traitement d'un cancer, ou
g) d'une dose thérapeutiquement efficace d'un antibiotique, ou
h) d'une dose thérapeutiquement efficace d'un composé antiviral, éventuellement le composé antiviral comprenant un inhibiteur de protéase, ou
i) d'une dose thérapeutiquement efficace d'un composé anticonvulsif, éventuellement le composé anticonvulsif comprenant du furosémide.

7. Préparation d'agent thérapeutique selon la revendication 6, dans laquelle les doses thérapeutiquement efficaces d'insuline, d'exénatide, de liraglutide, d'hormone de croissance et de parathormone sont dans la plage de :
a) pour l'insuline, environ 1 à 10 unités, éventuellement dans la plage de 2 à 4, 3 à 9, 4 à 9, 5 à 8 ou 6 à 7 unités, une unité étant l'équivalent biologique d'environ 45,5 µg d'insuline cristalline pure ;
b) pour l'exénatide, environ 1 à 10 µg, éventuellement dans la plage de 2 à 4, 4 à 6, 4 à 8 et 8 à 10 µg ;
c) pour le liraglutide, environ 1 à 2 mg, éventuellement dans la plage de 1,0 à 1,4, 1,2 à 1,6 et 1,2 à 1,8 mg ;
e) pour l'hormone de croissance, environ 0,1 à 4 mg, éventuellement dans la plage de 0,1 à 1, 1 à 4, 1 à 2 et 2 à 4 mg ; et
f) pour la parathormone, environ 1 à 40 µg, éventuellement dans la plage de 10 à 20, 20 à 30, 30 à 40 et 10 à 40 µg.

8. Dose thérapeutiquement efficace d'insuline selon la revendication 6 ou 7, dans laquelle l'insuline est humaine ou dérivée d'une voie synthétique.

9. Combinaison d'agents thérapeutiques selon la revendication 6, partie i), dans laquelle la dose thérapeutiquement efficace d'exénatide est dans la plage d'environ 1 à 10 µg et la dose thérapeutiquement efficace de métformine est dans la plage d'environ 1 à 3 g

10. Dispositif selon la revendication 1, dans lequel la capsule est formée à partir d'un matériau biodégradable de sorte à se dégrader en tout ou partie dans le tractus intestinal.

11. Dispositif selon la revendication 1 ou 10, dans lequel la capsule inclut un revêtement entérique ou autre destiné à la protection de la capsule contre les acides de l'estomac tout en permettant une biodégradation dans l'intestin grêle.

12. Dispositif selon la revendication 1, dans lequel l'actionneur comprend en outre un élément pouvant se dilater.

13. Dispositif selon la revendication 12, dans lequel l'élément pouvant se dilater comprend un ballon, le ballon incluant un premier compartiment et un second compartiment qui sont séparés par une soupape de séparation, un liquide étant disposé dans le premier compartiment et au moins un réactif étant disposé dans le second compartiment.

14. Dispositif selon la revendication 13, dans lequel la soupape de séparation est conçue pour se dégrader en réponse à un pH de l'intestin grêle de telle sorte que lors de la dégradation, la soupape s'ouvre.

15. Dispositif selon la revendication 13 ou 14, dans lequel lorsque la soupape s'ouvre, l'au moins un réactif se mélange dans le liquide pour produire un gaz afin de dilater le ballon et faire avancer l'élément de pénétration de tissu dans la paroi de la lumière.
